# EUROPEAN PATENT SPECIFICATION

(11) **EP 2 252 155 B1**
(45) Date of publication and mention of the grant of the patent: **27.01.2016**
(21) Application number: 09710419.4
(22) Date of filing: 13.02.2009
(51) Int. Cl.: A01N 65/00, A61K 8/49, A61K 8/97, A61Q 5/00, A61P 17/00

(54) **METHOD FOR MINIMIZING THE CYTOTOXICITY OF THE EFFECTIVE AGENT INHIBITING THE GROWTH OF MICRO-ORGANISMS AND FOR MAXIMIZING THE EFFECT**
VERFAHREN ZUR MINIMIERUNG DER CYTOTOXIZITÄT DES WIRKSTOFFS, DER DAS WACHSTUM VON MIKROORGANISMEN HEMMT, UND ZUM MAXIMIEREN DER WIRKSAMKEIT
PROCÉDÉ POUR RÉDUIRE LA CYTOTOXICITÉ DE L'AGENT ACTIF INHIBANT LA CROISSANCE DE MICRO-ORGANISMES ET POUR AUGMENTER SON EFFET

(30) Priority: 14.02.2008 FI 20080113
(43) Date of publication of application: 24.11.2010
(73) Proprietor: Oy Granula AB LTD, 48101 Kotka (FI)
(72) Inventor: AHLNÄS, Thomas, FI-48130 Kotka (FI)
(74) Representative: Berggren Oy Ab
(86) International application number: PCT/FI2009/050116
(87) International publication number: WO 2009/101262

(56) References cited:
- WO-A-2004/000304
- WO-A-2005/047423
- WO-A-2007/096088
- WO-A-2007/096089
- WO-A-2008/020112
- VALIMAA ET AL: "Antimicrobial and cytotoxic knotwood extracts and related pure compounds and their effects on food-associated microorganisms" INTERNATIONAL JOURNAL OF FOOD MICROBIOLOGY, ELSEVIER SCIENCE PUBLISHERS, AMSTERDAM, NL, vol. 115, no. 2, 12 March 2007 (2007-03-12), pages 235-243, XP005921921 ISSN: 0168-1605 cited in the application
- BJARNE HOLMBOM ET AL: "Knots in trees A new rich source of lignans" PHYTOCHEMISTRY REVIEWS, KLUWER, NL, vol. 2, no. 3, 1 October 2003 (2003-10-01), pages 331-340, XP008104241 ISSN: 1568-7767 [retrieved on 2004-11-08]

## Description

The invention relates to a method according to claim 1

In cosmetic and food industry compositions, in commercial solvents and detergents as well as in industrially employed solvents, it is often necessary to add anti-microbial agents, such as agents inhibiting the growth of microbes, and microbicidic agents. Anti-micro-organistic agents may themselves be cytotoxic, skin irritative or otherwise harmful to the system when getting into contact with the skin. Moreover, the currently used anti-micro-organistic agents are without exception synthetic, which means that their production is a complicated process.

The object of the invention is to eliminate the above described drawbacks of the prior art. Thus, the principal object of the invention is to realize such compositions or semi-finished compositions used in the production of said compositions, where the anti-micro-organistic agent contained by the compositions or their semi-finished composition is obtained from the nature, and where the undesirable properties are minimal. Undesirable properties are for example cytotoxicity, other physiological toxicity, skin irritation and tendency to form or induce free radicals on the skin, for instance as the substance is decomposed owing to the effect of sunlight. For instance phenoxyethanol (derivative of benzoic acid) is a relatively wide-scale synthetic microbicide and preservative, used generally in foodstuffs and cosmetic compositions, because it is effective against both yeasts, fungi and Gram-negative bacteria. However, its use is restricted, because it has been found to be physiologically toxic, allergenic and skin irritative, in addition to which it decomposes owing in sunlight, thus forming free radicals. The object of the invention is to reduce the use of generally used synthetic substances inhibiting microbial growth on a wide scale, or to completely replace said substances, by a corresponding substance obtained from natural sources, which substance causes minimal irritation to mammal skin and has a minimal cytotoxicity.

Here the term 'substances inhibiting microbial growth on a wide scale' particularly refers to a substance that inhibits the growth of both Gram-positive bacteria and Gram-negative bacteria as well as yeasts and fungi.

A further object of the invention is to reduce the harmful properties of another substance inhibiting microbial growth contained in a composition, such as a synthetic substance inhibiting microbial growth on a narrow scale, by a mixture with a good availability and low production expenses. One of the most significant harmful properties is the cytotoxicity of a substance inhibiting microbial growth on a narrow scale.

Yet another object of the invention is to realize cosmetic industry compositions or semi-finished compositions used for manufacturing these compositions, which composition or semi-finished composition contains as few anti-micro-organistic agents as possible.

### General description of the invention

More precisely, the invention relates to a method for producing a cosmetic composition, according to claim 1, said composition containing a widely anti-micro-organistically effective agent, a carrier agent such as solvent, with either hydrophobic or hydrophilic nature, selected according to the target of usage, as well as a possible surface active agent. Hence:
- in the composition, there is included carrier agent, possible auxiliary agents and possible surface active agent conventionally used in a cosmetic composition,
- in the composition, there is included a compound mixture with an anti-micro-organistic effect on a wide scale, which is obtained by pulverizing wood material from preferably two different wood species and/or by extracting the possibly pulverized wood material from two different wood species, so that said mixture contains at least two different compounds selected from among the following group: lignans according to general formulas IA and IB (Appendix), stilbenes according to general formula II (Appendix), juvabiones according to general formula III (Appendix), flavonoids according to general formula IV (Appendix) and betulin and its derivatives (betulinic acid, betuloinic acid or betulonic acid), said mixture also containing oligomers of said polyphenolic compounds providing, however, that the compound mixture contains lignans, particularly 7-hydroxymatairesinol or secoisolariciresinol roughly 50-99.9 wt%; stilbenes, particularly pinosylvin or its ester or ether derivatives roughly 0 -70 wt%; oligomers of lignans, stilbenes, juvabiones or flavonoids roughly 1-31 wt%; providing, however, that the mixture contains at least one compound, selected from among the following group: 7-hydroxymatairesinol, secoisolariciresinol, cycloisolariciresinol, anhydrosecoisolariciresinol, α-conidendrin, α-conidendric acid, isohydroxymatairesinol and stilbenes according to formula II, as well as their ether and ester derivatives and stereoisomers. In the composition, there is included said compound mixture 0.1-5 wt% of the total weight of said composition, providing, however, that with said content, the cytotoxicity of said compounds dissolved in ethanol with a HaCat cell culture after 24 incubations is lower than the cytotoxicity of 0.02 - 0.1 wt% BHT dissolved in ethanol in the same incubation conditions, preferably lower than the cytotoxicity of 0.01 - 0.05 wt% BHT dissolved in ethanol in the same incubation conditions, in addition to which the mixture within said content range has wide-scale anti-microbial effects against Gram- bacteria, Gram+ bacteria, yeast and fungi in the composition.

Advantageously the compound mixture obtained from two different wood species, such as pine and spruce species and contains 7-hydroxymatairesinol or secoisolariciresinol 50 - 99.9 wt%, stilbene, particularly pinosylvin 0.1-70 wt%, conidendrin 3-6 wt%, lariciresinol 4-7 wt%, liovile 2-5 wt%, or ester or ether derivatives of said stilbene or lignan compounds or their stereoisomers, and also oligomers of lignans, stilbenes, juvabiones or flavonoids 5 -8 wt%. However, owing to the relatively high cytotoxicity of stilbenes, a mixture of polyphenolic compounds obtained from pine and spruce species preferably contains pinosylvins roughly 0.1 - 20 wt%, if it is added in a foodstuff composition or cosmetic composition. Now the content of stilbenes or their ester or ether derivatives in the end product is roughly if 0.1 wt% at most, in case in the composition there is added 5 wt% mixed extract obtained from two different wood species, said mixed extract containing 10 wt% mixture of phenolic compounds.

Here the term stereoisomers refers to compound diastereomers and to mixtures of different diastereomers, to pure enantiomers and rasemic mixtures of enantiomers.

The invention is first of all based on the surprising discovery that when in a composition meant for a mammal or a composition that in general gets into skin contact with a mammal at some stage, the employed anti-micro-organistic effective agent is a mixture of phenolic compounds obtained from extracting and/or pulverizing natural wood material, which mixture is not essentially refined, said mixture has remarkably less harmful properties than the generally used wide-scale synthetic anti-micro-organistic agents currently used in compositions, such as previously mentioned phenoxyethanol. The phenolic compound mixture according to the invention does not cause skin irritation even with high contents, is physiologically well tolerated and has a low cytotoxicity.

Secondly, the invention is based on the surprising observation that sufficient attention has previously not been paid to the level of overall cytotoxicity of the effective agents in cosmetic materials, foodstuffs, animal feed and packing materials, but each effective agent to be included in the composition, such as anti micro-organistic agent, has been added to an extent which its maximum cytotoxicity by any means has allowed. With this observation in mind, the applicant has attempted to achieve compositions where the overall cytotoxicity of the different components of the effective agent is brought as low as possible, while the effect of the effective agent remains the same.

In the present invention, the optimizing of the overall cytotoxicity is realized by using, instead of, or in addition to, the first agent inhibiting the growth of micro-organisms contained in the composition, a compound mixture that is obtained by extracting and/or pulverizing and that is not essentially refined. The mutual ratio between the phenolic compounds contained by said compound mixture, as well as the compounds contained by the mixture, can change depending on the effects required in the target of usage. The mutual content of the compounds contained in the compound mixture, the compounds and the effect of the mixture, as well as its profile of influence are adjusted depending on the target of usage of the composition, so that by using the compound mixture, there can be achieved a desired anti-microbial effect, while the overall cytotoxicity of the compound mixture is as low as possible and remains within certain limits in comparison with the cytotoxicity of BHT. Compound mixture using can possible second inhibiting the growth of micro-organisms agent harmful properties such as its own cytotoxicity, reduced remarkably target of usage, such as on the skin or hairs of a mammal, for instance by reducing the quantity of the second agent inhibiting the growth of micro-organisms from the conventionally applied level.

The term 'cosmetic composition' here refers to compositions meant for the treatment of the skin, teeth, hair and body hair of mammals. In these, the carrier agent is semisolid material such as cream, gel or paste; solid material such as solid foam; heterogeneous material such as solid matter mixture; liquid material such as homogeneous solution or colloidal solution, such as dispersion or suspension, microemulsion, nanoemulsion, or a gaseous material such as aerosol or mist.

The manufacturing of a mixture containing phenolic compounds is simple, and the mixture is amply available from wood industry pulping processes, so that the replacing of the effective agent by said mixture also is economically beneficial. The compound mixture according to the invention also has several other advantageous properties that its individual components do not necessarily have, so that by using the mixture, new properties can be obtained in the manufactured compositions, or that the profile of influence of the anti-micro-organistic agent can be adjusted.

By means with the compound mixture according to the invention, there also is achieved the advantage that by using said compound mixture, antioxidative and free radical capturing properties can be obtained in cosmetic, so that these types of compounds do not need to be separately added in said compositions.

The compound mixture is advantageously an unrefined powder ground of wood material, or a compound mixture extracted from wood material in an extraction solution, said compound mixture forming a homogeneous solution, suspension or dispersion with the extraction solution. Even more preferably, the compound mixture is an unrefined lignan mixture and/or stilbene mixture placed in a liquid, said mixture containing at least two different lignan and/or stilbene compounds. The mixture is advantageously obtained by extracting wood material from two different wood species in an alcohol-based solution. Preferably the wood material is obtained from wood knot material or stemwood material from adjacent to knots. Here the term unrefined compound mixture, raw extract, refers to a mixture that is obtained by pulverizing and/or extracting wood or plant material, which mixture has thereafter not been subjected to any such chemical or physical cleaning operations by which one of the mixture compounds would be completely removed from the mixture. Typical cleaning operations in this sense are chromatography or crystallization of certain compounds from the solution. Instead, the mixture may be subjected to such chemical treatments by which the content of one of the mixture compounds is adjusted in relation to other compounds contained in the mixture. This kind of operation is for example extraction by which the mutual contents of the compounds contained in the solution are adjusted with respect to each other, but by which the compounds are not completely removed. The phenolic compounds contained in the mixture may also be transformed into simple derivatives such as their esters, or into completely other stilbene/lignan/flavonoid compounds, for example when the pH of the extract solution is changed. The mixture of phenolic compounds to be included in the composition according to the invention, which mixture has free radical capturing properties, contains preferably at least two different compounds selected from the following groups belonging to flavonoids, lignans, juvabiones, stilbenes or betulin or its derivatives:
Lignans:
   - matairesinol, hydroxymatairesinol, oxomatairesinol, didemethyl matairesinol, didemethyl matairesinol, isohydroxymatairesinol, epi-isohydroxymatairesinol and their stereoisomers, among which particularly let us point out hydroxymatairesinol stereoisomers 7S, 8R, 8'R-hydroxymatairesinol and 7R, 8R, 8'R-allohydroxymatairesinol, and their stereoisomers and ester or ether derivatives,
   - secoisolariciresinol, isolariciresinol, lariciresinol, pinoresinol, dimethyl secoisolariciresinol, 7-hydroxysecoisolariciresinol, cyclolariciresinol, cycloisolariciresinol and their stereoisomers as well as their ester or ether derivatives,
   - nortrachelogenin and its stereoisomers and ester or ether derivatives,
   - enterolactone and its stereoisomers and ester or ether derivatives,
   - conidendrin, α-conidendrin and their stereoisomers as well as ester or ether derivatives,
   - lignan A and its stereoisomers and ester or ether derivatives,
   - liovile and its stereoisomers and ester or ether derivatives;
Juvabiones:
   - juvabiones and their stereoisomers and ester or ether derivatives;
Stilbenes:
   - pinosylvin, dihydropinosylvin, pinosylvin monomethyl ether, dihydropinosylvin monomethyl ether, resveratrol, astringin, isorhapontine, and their stereoisomers and ester or ether derivatives;
Flavonoids:
   - pinosembrin, catechin, pinobanxin, kaempferol, dihydrokaempferol, taxifolin, naringenin, teracasidine, ketoteracasidine, isoteracasidine, melacasidine, isomelacasidine and their stereoisomers and ester or ether derivatives,
   - betulin, betulinic acid, betuloinic acid or betulonic acid and their stereoisomers and esterized forms,
   - as well as the glycosidized forms of these compound, and their oligomers such as trimers and tetramers. These oligomers are here called oligolignans in case they are oligomers of lignans, stilbenes or juvabiones; free lignans and stilbenes are dimers, having 2 phenylpropane units coupled together by beta-beta bonds, and their oligolignans have 3 - 6 phenyl propane units (C₆C₃) coupled together by beta-beta bonds.

In this connection let us point out that compounds called lignans are generally (poly)phenolic compounds obtained from wood and plants, having 2 phenyl propane units coupled together by beta-beta bonds (IUPAC, 2000), but in this application it has been considered necessary to distinguish lignans, stilbenes and juvabiones from each other owing to their different microbiological effects.

Lignans proper here refers to compounds according to the general formula IA and IB (Appendix).

Now, in formula IA:
R1 or R2 denote, irrespective of each other, residue selected from the groups hydrogen, OH or =O,
or either one of the residues R1 or R2 denotes the oxygen atom -O- bound to carbons 9 and 9', and now forms with carbons 8, 8', 9, 9' a 5-membered oxygenous heterocyclic ring C,
R3 denotes hydrogen or residue selected from the group OH, =O, or it forms a bond to the carbon 6, so that the carbons 6, 1, 7, 8, 8', 7' form a cyclohexane ring that is condensed with the phenyl ring A, and possibly also a ring with C,
R4 denotes hydrogen or methyl,
R5 denotes hydrogen or residue selected from the groups OH and OCH₃,
R6 denotes hydrogen or hydroxy,
R7 and R8 denote, irrespective of each other, hydrogen or residue selected from the groups OH and OCH₃.

Advantageous lignans according to formula 1A are:
7-hydroxymatairesinol (R1 denotes group =O, R2 denotes the oxygen atom pertaining to the hetero ring C, R4= CH₃, R7= OCH₃, R3=R5=R8=OH, R6=H, R9=H),
matairesinol (R1 denotes group =O, R2 denotes the oxygen atom pertaining to the hetero ring C, R3=R6=R9=H; R4= CH₃, R7= OCH₃, R5=R8=OH),
oxomatairesinol, which differs from hydroxymatairesinol in that R3 denotes group =O,
didemethyl matairesinol, which differs from hydroxymatairesinol in that R4 and R3 denote hydrogen,
isohydroxymatairesinol,
alpha-conidendrin (R1 denotes group =O, R2 denotes the oxygen atom pertaining to the hetero ring C, R4= CH₃, R7= OCH₃, R8=OH, R6=H, R3 is a bond to the carbon 6, R9=H),
alpha-conidendric acid (R4= CH₃, R7= OCH₃, R5=R8=OH, R6 denotes group =O, R1=R2=OH, R3 is a bond to the carbon 6, so that the carbons 6, 1, 7, 8, 8', 7' form a cyclohexane ring D that is condensed with the phenyl ring A, R9=H),
liovile (R4= CH₃, R7= OCH₃, R3=R5=R6=R8=OH, R1=H, R2 denotes the oxygen atom pertaining to the hetero ring C, R3 denotes a bond to the carbon 6, R9=H),
secoisolariciresinol (R1=R2= OH, R3=H, R4= CH₃, R7= OCH₃, R5=R8=OH, R6=R9=H),
dimethyl secoisolariciresinol, which differs from secoisolariciresinol in that R5 and R8 are methoxies,
isolariciresinol (R1=R2=OH, R4= CH₃, R7= OCH₃, R5=R8=OH, R6=H, R3 is a bond to the carbon 6, so that the carbons 6, 1, 7, 8, 8', 7' form a cyclohexane ring D, which is condensed with the phenyl ring A, R9=H),
cyclolariciresinol (R1=R2= OH, R3= a bond to the carbon 6, so that the carbons 6, 1, 7, 8, 8', 7' form a cyclohexane ring D, which is condensed with the phenyl ring A, H, R4= CH₃, R7= OCH₃, R5=R8=OH, R6=R9=H),
nortrachelogenin (R1 denotes group =O, R2 denotes the oxygen atom pertaining to the hetero ring C, R3=R6=H, R4=CH₃, R7=OCH₃, R5=R8=OH, R9=OH),

In Formula IB:
R10 denotes hydrogen or hydroxy
R11 denotes hydroxy or oxygen, which is bound by a bond to the carbons 7 and 9', forming now an oxygenous non-aromatic 5-membered heterocyclic ring (tetrahydrofuran) F with the carbons 7, 8, 8', 9', which ring is condensed in the hetero ring E (tetrahydrofuran) at the carbons 8, 8',
R12 denotes hydrogen or methyl,
R13 denotes hydrogen or methoxy,
R14 denotes hydrogen or methoxy,
R30 denotes hydrogen or group =O.

Advantageous compounds according to formula IB are:
pinoresinol (R13=R14= R12=R10=R30=H, and R11 is oxygen in the hetero ring F),
isohydroxymatairesinol (R12=R13=R14=R10=H, R11=OH, R30 denotes group =O),
lariciresinol (R12=R13=R14=R10=R30=H, R11=OH), and
lignan A (R10= R11=OH, R12=R13=R14=R30=H).
Stilbenes in turn refer to compounds according to the general formula II (Appendix),
where R15 denotes hydrogen or hydroxy
R16 denotes residue selected from the groups H, OH, OCH₃,
R17 denotes residue selected from the groups OH or OCH₃,
R18 and R19 denote, irrespective of each other, hydrogen or hydroxy,
R20 denotes residue selected from the groups hydrogen, OGlu
A few advantageous stilbenes according to formula II are pinosylvin (R18=R19=R20=R15=H, R16=R17=OH), monomethyl ether of pinosylvin (R18=R19=R20=R15=H, R16=OCH₃, R17=OH), dihydropinosylvin (R18=R19=R20=R15=H, R16=R17=OH, the phenyl elements bonding ethenyl residue is hydrated to ethyl), resveratrol (R16=R17=R19=OH, R18=R20=H), astringin (R15=R17=OH, R16=R19=H, R18=OH, R20=OGlu and isorhapontine (R16=R19=H, R17=OCH₃,R15, R18=OH, R20=OGlu).
Juvabiones refer to compounds according to formula III (Appendix).
Flavonoids refer to compounds according to general formula IV (Appendix), where
R21 denotes residue selected from the groups H, OH,
R22 denotes residue selected from the groups H, OH, =O,
R23 denotes residue selected from the groups H, OH
R24, R25, R26 denote, irrespective of each other, hydrogen or hydroxy,
R26 and R27 denote, irrespective of each other, hydrogen or hydroxy.

Among advantageous compounds according to formula IV are dihydromyricetin (R27=H, R21=R23=R24=R25=R26=R28=OH, R22 is oxo group), taxifolin (R24=R27=H, R21=R23=R25=R26=R28=OH, R22 is oxo group), dihydrokaempferol (R24=R26=R27=H, R21=R23=R25=R28=OH, R22 is oxo group), catechin (R24=R26=R27=H, R21=R23=R25=R26=R28=OH, R22 is hydrogen), naringenin (R23=R24=R26=R27=H, R21=R25=R28=OH, R22 is oxo group), kaempferol (R24=R26=R27=H, R21=R23=R25=R28=OH, R22 is oxo group), teracasidine (R21=R24=H, R22=R23=R25=R26=R27=R28=OH, ketoteracasidine (R21=R24=R26=H, R23=R25=R27=R28=OH, R22 is oxo group), isoteracasidine (R21=R24=R26=H, R22=R23=R25=R27=R28=OH), melacasidine (R21=R24= H, R22=R23=R25=R26=R27=R28=OH), isomelacasidine (R21=R24=H, R22=R23=R25=R26=R27=R28=OH), pinobanxin (R24=R25=R26=R27=H, R21=R23=R28=OH, R22 is oxo group) and pinosembrin (R23=R24=R25=R26=R27=H, R21=R28=OH, R22 is oxo group).

Betulin, by systematic name (IUPAC) lup-20(29)-ene-3,28-diol, and its derivatives refer to compounds according to formula 1E. In the formula IE, betulonic acid is compound 2, and betulinic acid is compound 3. Betulin is compound 1. Compounds 2 and 3 are obtained by oxidizing betulin 1 into compound 2, and by reducing compound 2 into compound 3 (US 6.280.778). Betuloinic acid is a derivative of betulonic acid.

Exemplary oligolignans are beta bound guaiacyl ethers of lignans and stilbenes (trimeric 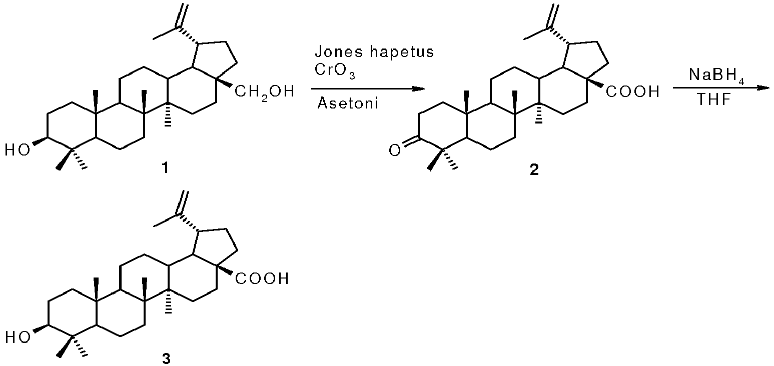 so-called sesquilignans) and coumarates such as secoisolariciresinol guaiacyl glycerol ether, nortrachelogenin guaiacyl glycerol ether, hydroxymatairesinol guaiacyl glycerol ether, lariciresinol guaiacyl glycerol ether, liovile guaiacyl glycerol ethers, conidendrin guaiacyl glycerol ether, pinoresinol guaiacyl glycerol ether, lariciresinol coumarate and secoisolariciresinol coumarate (Willför et al, Holzforchnung, Vol 58, 3435 -354, 2004) and dilignans such as 5-5-bis-secoisolariciresinol, 5-5-bis-isolariciresinol, 5-5-bis-lariciresinol.

Depending on the pH, phenolic compounds occur in the compositions either as free, esterized or etherized forms, wherefore also said ester and ether derivatives belong within the scope of the invention.

Free radical here refers to a molecule or an atom that has unpaired electrons in its electron shell. Typical free radicals are oxygen radical, hydroxyl radical and peroxyl radicals as well as superoxide radicals, but also for example lipid radicals. Further, for instance titanium oxide can be formed into a reactive free radical, in case it is tuned to a higher energy state owing to the effect of UV radiation. A compound is called free radical capturer, in case it is capable of inhibiting the creation of free radicals, or their function. In case the compound is capable of inhibiting the creation of particularly oxygenous free radicals and their effects, the compound is called antioxidant.

One of the most important properties of a compound mixture according to the invention is that the mixture of phenolic compounds present in the compositions inhibits on a wide scale the growth of anti-microbial agents, when it is added in the composition for 0.1-5 wt%. Generally the amount of synthetic widely anti-microbial agents that can be added to compositions is only roughly 0.01-0.03 wt%, owing to their high cytotoxicity, in case the compositions get into contact with mammal skin at some stage of their usage life. As for Gram-negative bacteria, a mixture of phenolic compounds has a growth inhibiting effect is at least against E. coli, Ps. aeruginosa, Ps. putida, and Kl. pneimoniae; as for Gram-positive bacteria, it has a growth-inhibiting effect at least against S. aureus; as for yeasts, it has a growth-inhibiting effect at least against M. furfur and C. albicans, and as for fungi, a growth-inhibiting effect at least against A. niger.

Thus, most of the compound mixtures according to the invention are relatively mild anti-micro-organistic agents, but this is compensated in that owing to their non-toxic nature, they can be used in remarkably larger quantities.

A particular surprising feature in the invention is that although it has been found out that several pure lignan or flavonoid compounds, or knot extracts obtained from trees containing abundantly such lignans or flavonoids, have a limited effect against the growth of micro-organisms, yeasts or fungi, it has not been verified that they should have a wide-scale anti-micro-organistic effect, and they are not effective for example against certain important Gram-positive bacteria such as S. aureus (cf. e.g. Välimaa et al., International J. of Food Microbiology, 115 (2007) 235-243). Moreover, it has earlier been shown that stilbene-bearing raw extracts and solutions containing refined stilbenes are relatively cytotoxic (e.g. International J. Food Microbiology, 115 (2007) 235-243), which does not encourage a man skilled in the art to use these extracts in cosmetic products. The low cytotoxicity of the mixtures according to the invention, combined with low skin irritation and a wide-scale anti-micro-organistic effect, guarantees that the compound mixture according to the invention can be used in sufficiently large quantities, particularly in cosmetic compositions, in order to ensure its anti-microbial effect. The wide-scale anti-micro-organistic effect of the mixtures containing phenolic compounds used in the invention, combined with their low cytotoxicity, is a surprising feature, because in the raw extracts obtained from knotty stemwood described in the prior art, their anti-micro-organistic effect has not been verified as particularly wide-scale, not even for raw extracts containing stilbenes.

Further, one of the most important properties of the compound mixtures according to the invention is their minimal penetration to skin, wherefore skin irritation does not occur when using them, as opposite to the conventionally used anti-micro-organistic compounds. The applicant has studied the skin irritation of the compound mixtures according to the invention in a so-called single patch test and found out that they do not irritate the skin with 0.1 wt% contents, and not even with 1 wt% contents; often even 5 wt% contents can be used without excessive skin irritation.

By using the microbial growth inhibiting compound mixture according to the invention, it is often possible to modify the harmful properties of other anti-micro-organistic agents, such as microbicidic and bacteriostatic agents contained in the composition, by reducing the among of free radicals created during their decomposition, and hence the cytotoxicity of these agents.

In an embodiment of the invention, in the composition to be prepared there are not included other anti-microbial agents except those present in the compound mixture obtained from wood material.

Among others, the anti-micro-organistic compound mixture according to the invention can be used in cosmetic compositions and their semi-finished.

### Description of preferred embodiments of invention and of compound mixtures used therein

One of the advantageous uses of the compound mixture according to the invention is a semi-finished product used in the manufacturing of cosmetic compositions, which is formed of alcohol solvent used as the carrier, and of an anti-micro-organistic mixture according to the invention. Several examples of this embodiment are given in the description below.

Primarily the effective free radical capturing mechanism of the (poly)phenolic compounds contained in the compound mixture according to the invention appears to be based on the fact that mixture the phenolic compounds contained by the mixture go through an automatic oxidization-reduction reaction after neutralizing the free radicals. This automatic oxidization-reduction reaction ends in the formation of stabile dimers. Polyphenol dimers can thereafter be split back to polyphenols (regeneration). A secondary effect of the mixtures according to the invention lies in that they are capable of absorbing the UV radiation that creates free radicals, and inhibiting the action of enzymes creating free radicals and/or inhibiting the action of metal ions catalyzing the formation of free radicals, or breaking up hyperoxides. The phenolic compounds contained in the compound mixture according to the invention are well tolerated by mammals, their cytotoxicity is low and they do not themselves form nor induce free radicals.

The production method of a compound mixture according to the invention, and the phenolic compounds contained therein, as well as the mutual ratios of their quantities depend on the designed usage and on the availability of raw materials. The mixture according to the invention is often obtained from the material of two or more wood species. Thus for example wood processing industry generally uses both spruce and pine in the pulping process. The production of a mixture according to the invention can utilize knot wood or knotty stemwood parts that are less suitable in the pulping process. However, because the pulping process mainly uses spruce and to a lesser amount pine, it is often more advantageous to form a compound mixture of spruce, which is more disadvantageous for the use of the composition, because it has better availability than pine. For example, a compound mixture obtained from pine wood knot material in hydrophilic extraction has a remarkably abundant quantity of stilbenes, which are effective microbicides and effective compounds inhibiting microbial growth. As for a compound mixture obtained from spruce wood knot material in a hydrophilic extraction, it contains a remarkable quantity of 7-hydroxymatairesinol, as well as its isomers and derivatives, such as matairesinol. Although the microbicidic efficiency of 7-hydroxymatairesinol is lower than that of stilbenes, particularly that of pinosylvin and its derivatives, it is profitable to use knot extract obtained from spruce, which has better availability and thus generally lower price, in the microbicidic compound mixture.

The phenolic compound mixture according to the invention is produced either by pulverizing or by extracting wood material, or by combining said procedures. Because the wood material most widely available in Finland is pine or spruce, of which particularly the latter contains resin, the extraction is generally realized in two steps. Now resin compounds are extracted from the pulverized pine material, advantageously pulverized knot wood or knotty stemwood material, by a lipophilic extraction solvent, and successively the polyphenols are extracted by a hydrophilic extraction solution. The lipophilic organic extraction solution is for example hydrocarbon, such as lower alkane, for example hexane or heptane. Generally the hydrophilic organic extraction solution is an organic compound containing a carbonyl group, such as alcohol or ketone. Ketone can be used only in case the compound mixture is meant for technical usage. An advantageous ketone is a lower alkyl ketone such as acetone. In case the compound mixture to be produced is a compound mixture in pulverized form, water is removed from the pulverized knotty stemwood material by freeze-drying.

Advantageously the alcohol is a monovalent, bivalent or trivalent lower alkyl alcohol, or a mixture of these. The monovalent lower alkyl is preferably ethanol, propanol, butanol, heptanol, octanol or decanol. A mixture of a lower alkyl alcohol and glycerol or glycol is an advantageous solvent agent when producing several skin care compositions or semi-finished products of skin care compositions, where the carrier agent is liquid. As for the lower alkylene glycol used as the hydrophilic extraction solution, it is preferably selected from a group comprising propylene glycol, butylene glycol, pentylene glycol and dipropylene glycol. Of these, the latter is particularly advantageous to be used in perfumes. In this kind of extract solution or in an extract concentrate obtained therefrom, the content of alkylene glycol is more than 7 wt%, preferably more than 10 wt%.

The composition of a compound mixture containing phenolic compounds, obtained from one and the same wood material by different extraction methods, fluctuates to some extent. The extraction method is selected according to the target of usage of the composition (for instance use in commercial solvents or cosmetic compositions), and according to the desired anti-micro-organistic properties of the compound mixture. For example from Table 2 to be described below, it can be observed the total quantities of polyphenolic lignan and stilbene compounds contained in pine knotwood and obtained by various extraction methods, as well as the mutual ratios of said polyphenolic compounds, fluctuated to some extent. Moreover, the obtained extract contained a certain amount of resin elements.

In case the extraction methods are changed, lignans can be transformed to other lignans. For example, 7-hydroxymatairesinol can in alkaline extraction conditions be transformed to alpha-conidenrin and further to alpha-conidendric acid or 7-hydroxymatairesinolic acid, and further to isohydroxymatairesinol. In acidic extraction conditions, 7-hydroxymatairesinol is transformed to isohydroxymatairesinol, and alpha-conidendric acid in turn is transformed to cyclolariciresinol, and secoisolariciresinol is in acidic conditions transformed to anhydrosecoisolariciresinol.

Hardwood species contain remarkable quantities of different flavonoids, biflavonoids and flavonoid glycosides, as is apparent from Table 3A below. Unrefined raw extracts containing flavonoids, obtained from hardwood species, have antioxidative and free radical capturing properties, wherefore they can be used for example together with raw extracts obtained from knotty pine or spruce stemwood for producing compound mixtures containing phenolic compounds according to the invention, as well as their intermediate products.

It has been found out that an unrefined extract solution obtained from knotty spruce stemwood material by hydrophilic extraction has, in its spectrum of influence, a similar wide-scale microbial growth-inhibiting effect as extracts obtained from knotty pine stemwood (cf. Table 4B). A raw extract extracted from spruce knotwood in alcohol contains mainly lignans and oligolignans (cf. Table 3B below). Lignans contain mainly hydroxymatairesinol, secoisolariciresinol, conidendrin and oligolignans, as well as smaller amounts of other lignans such as liovile and lariciresinol. Although the microbial growth inhibiting effect of polyphenolic lignan compounds contained in a raw extract obtained from knotty spruce stemwood by alcohol is weaker than with raw extracts obtained from knotty pine stemwood (cf. Table 4B below), said extracts obtained from spruce stemwood chips by hydrophilic extraction can be used for producing cosmetic compositions according to the invention and their semi-finished products owing to their low cytotoxic effect and low skin irritation.

As can be observed for example from Table 4A below, extracts obtained from the bark of different wood species by hydrophilic extraction contain various polyphenolic flavonoid compounds. In the present invention, these unrefined extracts mainly containing flavonoids can be used either as such or preferably together with hydrophilic extracts obtained from other the material of wood species, to be further used for producing various compositions according to the invention and their semi-finished products. One source of these advantageous flavonoids is the bark of birch (Pendula betula). Compounds isolated from birch bark are enlisted in Table 4A and in Appendix 3 Figure 2 illustrates compounds isolated from birch bark. As can be observed from Figure 2 of Appendix 3, birch bark contains lignans, stilbenes, flavonoids, juvabiones and betuligenol and its derivatives, wherefore it can also be used as a raw material for producing an anti-micro-organistic compound mixture according to the invention. Betuligenol (betulin) mentioned at the end of Figure 2 of Appendix 3, as well as its derivatives betulinic acid, betuloinic acid or betulonic acid, can also be used in compound mixtures according to the invention, either as separately added therein in refined form, or together with an unrefined compound mixture obtained from birch bark extraction.

In Figure 1 illustrated in Appendices 1 and 2, there are introduced polyphenolic compounds present in pine. As is apparent from Figure 1 of Appendices 1 and 2, as well as from Table 4B, knotty pine stemwood and its bark contain several different stilbene compounds. Moreover, the percentual share of these stilbene compounds in the pine polyphenolic compounds present in knotty stemwood and bark is remarkably high in comparison with the quantity of other polyphenolic compounds. Further, pine contains a remarkable quantity of various flavonoids.

Unrefined extracts obtained from knotty pine stemwood by hydrophilic extraction contain a remarkable quantity of stilbene compounds. The applicant has verified that these extracts inhibit the growth of micro-organisms (Gram-positive and negative bacteria, fungi and yeasts) in a wide scale. Thus, pine is a good source of the anti-micro-organistic compound mixtures according to the invention. It has also been discovered that stilbenes have antiinflammatory properties. On the other hand, unrefined raw extracts obtained from spruce contain mainly lignans; the applicant has verified that said lignans have, by their spectrum of influence, a similar but weaker effect for inhibiting the growth of micro-organisms than stilbene-bearing raw extracts obtained from pine. Both raw extracts extracted from knotty pine stemwood and containing mainly stilbenes, as well as raw extracts extracted from knotty spruce stemwood and containing mainly lignans, are feasible when manufacturing different compositions that have a wide-scale anti-microbial effect and at the same time low skin irritation and low cytotoxicity in comparison with BHT.

From the pulping processes of wood processing industry, there is obtained remarkably more spruce than pine, which fact is in favor of a solution that the compound mixtures according to the invention comprise compound mixtures of unrefined extraction solutions obtained from spruce by hydrophilic extraction, or unrefined extraction solutions obtained from spruce and pine by hydrophilic extraction. In a suitable arrangement, the combined extraction solutions according to the invention contain for example 70 wt% (poly)phenolic compounds (lignans and oligolignans) extracted from knotty spruce stemwood, and 30 wt% (poly)phenolic compounds extracted from knotty pine stemwood, with stilbenes included. Other mixture ratios can also be applied, as long as it is taken care of that the compound mixture contained in the combined extraction solution obtained from pine and spruce has a sufficiently low cytotoxicity (the employed reference is BHT). The applicant has discovered that when the mutual mixing ratios of the raw extracts obtained from different wood species are selected so that the cytotoxicity of the compound mixture contained in the combined raw extract, when measured in ethanol for a HaCat cell culture after 24 incubations, is lower than the cytotoxicity of 0.02 -0.1 wt% BHT dissolved in ethanol in the same incubation conditions, preferably lower than the cytotoxicity of 0.01 - 0.05 wt% BHT dissolved in ethanol in the same incubation conditions, the allowed quantity for the employed mixture is 0.1 -5 wt% of the total weight of the composition, in most cases 1 - 5 wt% of the total weight of the composition. This limit value of the content is remarkably higher than with most commercially available wide-scale, microbial growth inhibiting synthetic substances, which means that the compound mixtures according to the invention can be used in sufficient quantities for ensuring their wide-scale anti-micro-organistic effect.

In Tables 5A, 5B, 5C and 5D included in Appendix, there are represented antioxidative and free radical capturing effects of pure polyphenolic compounds and of unrefined polyphenolic compounds containing raw extracts obtained from wood bark and knotty stemwood.

Tables 5A and 5B represent the antioxidative effect of a few pure polyphenols and of unrefined polyphenolic compounds containing raw extracts, obtained from wood bark and knotty stemwood. Tables 5C and 5D in turn illustrate the peroxide radicals capturing capacity of a few pure polyphenols and of unrefined, polyphenolic compounds containing raw extracts, obtained from wood bark and knotty stemwood. In Tables 5A-5D, it can be observed that the antioxidative and free radical capturing properties of solutions containing pure polyphenol compounds, obtained from wood material in hydrophilic extraction, are often remarkably different from the corresponding properties of compound mixtures containing unrefined raw extract solutions and obtained from wood material in hydrophilic extraction, owing to the synergetic effects of the compounds contained in the compound mixtures in unrefined extracts. The compound mixtures according to the invention are obtained from these unrefined raw extract solutions.

### Production of the compositions and their semi-finished products

A phenolic compounds containing mixture according to the invention can be included in cosmetic compositions and their semi-finished products in a way known as such, of which examples are also given below. Thus, in case a compound mixture is extracted for instance of wood material by an alcoholic solution into a raw extract, this raw extract can be made into a homogeneous mixture such as a homogeneous solution, or a colloidal dispersion with two or several phases such as gel, paste, emulsion, microemulsion, nanoemulsion suspension, dispersion or mist. In that case a phenolic compounds containing raw extract is included in a homogeneous solution by dissolving, and/or it is included by dispersing to the carrier agents of a colloidal mixture in a way known as such, so that the raw extract is admixtured either in a phase containing a continuous carrier agent, or to a phase containing a carrier agent to be dispersed. For forming a carrier of carrier agents, there are is used conventional auxiliary agents of the trade. Such agents are, among others, surface active agents, dispersing agents such as emulsifying agents, gel formers such as carbomers and methylcellulose.

The employed carrier agents are gel base formers such as water or alcohol, cream base and paste base formers such as paraffins, waxes, silicones, aqueous phase forming agents (water) or phase formers such as paraffin or stearic acid. The compositions can also be multi-phase compositions, so that the carrier agent is formed of several aqueous and/or oil phases. With respect to the manufacturing of various compositions, we refer to the literature of the trade /1/, /2/, /3/ and to the examples to be given below.

In these homogeneous solutions and colloids, there can be admixtured additives such as UV protective agents, antioxidants and vitamins, surface active agents, moisturizing agents, moisture maintaining agents, stabilizing agents, moisture absorbing agents, emollients, fats, lubricants, perfumes, viscosity regulators, colorants, antioxidants and narrow-scale anti-microbial agents etc., in a conventional way known as such, with respect to which we refer to the literature of the trade /1/, /2/, /3/.

Surface active agents applicable in exemplary liquid compositions according to the invention, and in their semi-finished compositions are: tensides, lecithin, caprylic acid and monoglycerides and diglycerides of capric acids, polyglyceryl-3-di-isostearate/polyglyceryl-2 and polyhydroxystearate, alkyl glycoside/alkyl alcohol, cetearyl pyridium chloride, bentsalkonium chloride, ionogenic agents, cetearyl glycosides, lower alcoxilated glycosides and micelle-forming agents.

Perfumes can be selected for example from a group including phenyl ethyl glycol, eugenol, isoeugenol, geraniol, citronellol or linalool, or their esterized forms or their aldehydes. Colorants can be selected for example from among the colorants accepted by FDA to be used in foodstuffs and cosmetic products.

In compositions according to the invention, as well as in their semi-finished products, it is also possible to add other effective agents as additives. Such effective agents to be used as additives are for example antioxidants. Among antioxidants, let us point out natural and synthetic vitamins such as vitamin A, B, C. D, E, provitamin B5, vitamin B3, L-ascorbic acid and vitamin E; further, there can be used antioxidants obtained from natural sources, such as antioxidants contained in green tea, antioxidants contained in flaxseed, antioxidants contained in horse chestnut, beta carotene, selenium, glutamine, ubiquinone (coenzyme Q10), glycolic acid, growth hormones and kinetin.

An advantageous botanical microbial growth inhibiting agent is betulinic acid, betuloinic acid or betulonic acid (US 6 280 778), derivatives of betuligenolin, and resvatrol obtained from spruce bark. These have been found to have an anti-microbial effect, and their cytotoxicity for healthy cells is low, and they enhance the dying of cancer cells. These can be added, either as pure compounds or as unrefined extract solutions obtained from wood bark, or as powders, in compositions and semi-finished products to be manufactured according to the invention.

Further, in compositions to be manufactured according to the invention, there can also be added pure flavonoids, lignans and stilbenes as well as their oligomers isolated from plants. In this application, the term 'oligomers' refers to homologs of a compound, i.e. to its dimers, trimers etc., where the included number of similar units is lower than in a polymer. Suitable botanical polyphenol compound sources are oilseeds, nuts, grain, fruits, berries and pulses.

One semi-finished product used in the method according to claim 1, contains, as a carrier, a solvent system with either hydrophobic or hydrophilic nature, selected according to the target of usage, a compound mixture to be included in the composition, and a possible dispersing agent. Sais semi-finished composition is a concentrate, in which there is added a solvent, such as water, for producing the end composition. Preferably this semi-finished product contains, as the carrier agent, alcohol in which there is admixtured a narrow-scale anti micro-organistic agent such as paraben, a mixture of polyphenolic agents to be included in the end composition, short-chain ester/ether and possibly UV protective agent. More preferably this semi-finished product contains, as the carrier agent, water and alcohol and as an auxiliary agent nonionic surface active agent.

### Examples

### Field tests

### A) Cytotoxicity in comparison with BHT

Table 1 shows comparisons between the cytotoxicity of a compound mixture according to the invention and the cytotoxicity of an antioxidant (BHT) that is widely used in food and cosmetic industry.

**Table 1**

| | | |
|---|---|---|
| The cytotoxicity of sample extracts pulverized or alcohol-extracted of knotty spruce stemwood chips, containing the compound mixture, or a powder containing the compound mixture, in comparison with the cytotoxicity of BHT (butylated hydroxytoluene). The composition of the individual compounds included in the compound mixture contained by the samples was in accordance with Table 3B. The cytotoxicity of the sample extracts and powders for human keratinocyte cells was measured. The employed measure of cytotoxicity was the total protein quantity created by the samples, when the samples were incubated together with human keratinocyte cells for a certain incubation time (24 h). For each sample, there was searched a limit value (EC20), by which 20% of the cultivated cells died. | | |

| Extraction solvent | sample | EC20 ppm 24 h |
|---|---|---|
| pentylene glycol | HMR-5 | 450 |
| butylene glycol | HMR-4 | 600 |
| glycerol | HMR | 1.260 |
| | HMR powder | 160 |
| with propylene glycol | HMR-3 | 620 |
| ethanol | HMR extract | 550 |
| ethanol | BTH | 5.50 |

From Table 1 it is observed that the most cytotoxic substance was BTH, which was 10-20 times more cytotoxic than HMR powder. HMR powder was obtained by pulverizing knotty spruce stemwood chips without other further cleaning, and said powder contained mainly 7-hydroxymatairesinol as well as, to a lesser degree, other polyphenolic lignans. Other knot extracts obtained from spruce knotwood chips by hydrophilic extraction with alcohol (ethanol, propylene glycol, pentylene glycol, butylene glycol, or glycerol) also contained lignan mixtures according to the invention, which included, as their principal component, 7-hydroxymatairesinol and also other phenolic lignans. With respect to HMR extract mixtures, BTH was 50-100 times more cytotoxic.

### B) Anti-microbial effect

### Test 1

The anti-micro-organistic effect of a few compound mixtures according to the invention against bacteria, yeast and fungi was examined:
- raw extracts extracted from knotty spruce stemwood chips by 4-glycol (sample 1), 5-glycol (sample 2) and 3), glycerine (sample 3) and glycerol (sample 4), containing 10 wt% of the compound mixture, the composition of the polyphenolic compounds of said raw extracts being in accordance with Table 3B (contained most 7-hydroxymatairesinol).
- combined raw extract (sample 5), extracted of knotty spruce stemwood chips and knotty pine stemwood by ethanol (pine) and by butylene glycol (spruce), containing roughly 10 wt% of the compound mixture obtained from pine and spruce. The sample mixture contained both a lignan mixture obtained from Norway spruce (Picea abies), the composition of which was in accordance with Table 3B, and a mixture of lignans and stilbenes obtained from pine (Pinus sylvestris), the composition of which roughly corresponded to the one illustrated in Table 2 (test 2, ethanol extraction).

The growth-inhibiting effect of the mixture samples was verified against the following micro-organisms:
Staphylococcus aureus ATCC 6538
Esterichia coli ATCC 8739
Pseudamonas auriginosa ATCC 9027
Psudomonas putida ATCC 49128
Klebsiella pneumoniae ATCC 10031
Candida albicans ATCC 10231
Malassezia furfur ATCC 96809 (yeast fungus)
Aspergillus niger ATCC 16404

### Performance of the study

In each of the 10 g sample batches taken from the samples, there were added different microbe cell suspensions, where the microbe population density was at least 5 x 10⁶ microbes/ml. The sample batches were incubated at room temperature (22 °C) for 2, 4, 24 and 48 hours, 4 days, 7 days, 14 days and 28 days.

When reproductive microbes were not found in the samples anymore, each sample was cultivated for a 1 ml sample batch in a 100 ml Letheen broth base, and after concentration, possible bacterial growth was further checked on a culture base.

### Results

All samples had an extremely good anti-microbial effect; after an incubation time of 4 hours, 24 hours,48 hours, 7 days, 14 days and 28 days, microbial growth was not detected.

Both the raw extracts obtained from spruce material according to the invention by alcohol extraction, and the raw extracts obtained from pine material by alcohol extraction, as well as the raw extracts obtained from a combined spruce and pine material by alcohol extraction prevented the growth of anti-microbial agents on a wide scale. They prevented the growth of both Gram-negative bacteria E. coli, Ps. aeruginosa, Ps. putida, Kl. pneimoniae) and Gram-positive bacteria (S. aureus). In addition, they also efficiently prevent the growth of yeasts (M. furfur, C. albicans) and fungi (A. niger).

### Test 2

Raw extract extracted from knotty spruce stemwood with pentylene glycol, containing a 3 wt% mixture of phenolic compounds, the composition of which was in accordance with Table 3B, was added in a sun protection cream for 0.3 wt% and 0.5 wt%, as well as known ethylhexyl glycerine (EH), efficient against Gram-positive bacteria. For the sake of comparison, to the same sun protection creams there was added a conventionally used, widely anti micro-organistic agent, phenoxyethanol (FE), which has a relatively low highest acceptable quantity of usage owing to its cytotoxicity and skin irritative properties.

| Test | number of inoculation cycles |
|---|---|
| | 01 23456 |
| Test 1 | |
| cream base | --- +Y +M,Y +M,Y ++M,Y |
| 0.3 wt% EH | |
| 1 wt% FE | |
| Test 2 | ------- |
| Cream base | |
| 0.5 wt% FE | |
| 1 wt% EF | |
| Test 3 | ------- |
| Cream base | |
| 0.3 wt% EH | |
| 3 wt% HMR-5 | |
| Test 4 | ------- |
| Cream base | |
| 0.3 5-% EH | |
| 3 wt% HMR-5 | |

| | |
|---|---|
| Y= yeast growth M= Fungal growth - no microbial growth + slight microbial growth ++ moderate microbial growth | |

As is apparent from the above described test 2, when a 3 wt% raw extract obtained from knotty spruce stemwood was used in a sun protection cream instead of phenoxyethanol, it was possible to maintain a wide-scale anti-microbial effect. The effect was maintained, although the content ethylhexyl glycerine, inhibiting the growth of Gram-positive bacteria, was reduced, which shows that the raw extract from knotty spruce stemwood itself has a Gram-positive bacteria growth-inhibiting effect.

The sun protection cream used in the above described test was a cream according to example 2. When using phenoxyethanol, the highest acceptable content of which is 1 wt%, there was detected a growth of yeasts and fungi with an ethylhexyl glycerine content of 0.3% in the sun protection cream. On the other hand, with an ethylhexyl glycerine content of 0.5%, the growth of yeasts and fungi was inhibited. When phenoxyethanol was replaced by a lignan mixture HMR-5 obtained from knotty spruce stemwood with hydrophilic extraction (pentylene glycol), it was observed that the ethylhexyl glycerine content could be reduced to 0.3%, and yet the microbial growth in the cream was inhibited. Thus the alcohol extract (HMR-5) according to the invention, which contains a physiologically well-tolerated mixture, can be used to replace the anti-micro-organistic phenoxyethanol, the use of which is restricted owing to its physiological toxicity. The composition HMR-5 was in accordance with the composition according to Table 3B.

### C) extraction of phenolic compounds from wood material

**Table 2**

| Table 2 illustrates extracts obtained by different extraction methods from pulverized knotty stemwood material of pine (scots pine). | | | | |
|---|---|---|---|---|
| | Test 1 | Test 2 | Test 3 | Test 4 |
| Compound | % of peak | % of peak | % of peak | % of peak |
| PSMME | 16 | 17 | 23 (14) | 29(16) |
| PS | 15 | 20 | 19 (12) | 20(12) |
| NTG | 16 | 30 | 30(18) | 33(19) |
| Resin acids | 18 | 16 | 12(7) | 10(6) |
| Oxidized resin acids | 35 | 17 | 17 (10) | 8(5) |

In all tests, there were extracted pine chips composed of knotty stemwood. These were first extracted with hexane; in test 4, a technical hexane was used. After the extraction of lipophilic hexane, the samples were extracted with various hydrophilic solutions: in test 1 with acetone, in test 2 with ethanol (96%), in test 3 with acetone and in test 4 again with 96% ethanol. In tests 1 and 4, there were used chips were dead and live wood material were mixed; in tests 2 and 3, the employed wood material consisted of hand-picked dead knotty stemwood chips. After extraction, the quantities of the phenolic and resin compounds contained by the samples were analyzed by liquid-gas-chromatography. The contents of various compounds are given as a percentual area of the peak shown by each compound in relation to the area of all peaks. In tests 3 and 4, the weights of different compounds are given in parentheses with respect to the total weight of the solution.

Abbreviations of the compounds in the Table: PSMME: pinosylvin monomethyl ether (stilbene); PS: pinosylvin (stilbene); NTG: nortrachelogenin (lignan).

It has been discovered that raw extracts from pine knotwood are both microbial growth inhibiting on a wide scale, and also anti-inflammatory, obviously owing to the stilbene compounds contained therein, such as pinosylvin and its derivatives. Thus for example knot extracts containing unrefined phenolic compounds according to Table 2, from tests 1 and 3, can be used as such in commercial solvents. The pine knot extract obtained from examples 2 and 4, could in turn be used as such as a semi-finished product for manufacturing various cosmetic compositions, without further cleaning, in case the anti-micro-organistic compounds used in said products should be replaced by a mixture containing physiologically better tolerated phenolic compounds.

### D) polyphenolic compounds contained by different wood species

Mixtures according to the invention can be isolated from wood material in general. An advantageous raw material source consists of branches, knotwood and knotty stemwood, but also other wood parts, such as stemwood, wood bark and needles can be used.

**Table 3A**

| The principal components ofunrefined extraction solutionsobtained by hydrophilic extraction from knotty stemwoodof various hardwood species. | | | |
|---|---|---|---|
| **Acacia crassicarpa** | | **Populus grandidentata** | |
| Flavonoids | 54% | Flavonoids | 31% |
| Melacasidine | 24% | Dihydrokaempferol | 13% |
| Isomelacasidine | 18% | Catechin | 9% |
| Biflavonoids | 9% | Naringenin | 7% |
| | | Taxifolin | 3% |
| | | Flavonoid glycosides | 34% |

| **Acacia mangium** | | **Populus tremula** | |
|---|---|---|---|
| Flavonoids | 36% | Flavonoids | 21% |
| Teracasidine | 25% | Dihydrokaempferol | 17% |
| Ketoteracasidine | 3% | Naringenin | 3% |
| Biflavonoids | 8% | Flavonoids glycosides | 8% |
| | | | |

| **Fagus sylvatica** | | **Populus tremuloides** | |
|---|---|---|---|
| Flavonoids | 7% | Flavonoids | 23% |
| Catechin | 6% | Dihydrokaempferol | 17% |
| | | Naringenin | 10% |
| **Eucalyptus globulus** | | Kaempferol | 1% |
| Tannins | 19% | Taxifolin | 1% |
| Tannin monemers | 5% | Flavonoid glycosides | 24% |
| Ellagic acid | 3% | | |
| Gallic acid | 2% | | |

**Table 3B**

| Polyphenolic compounds contained in a solution extracted from Norway spruce knotty stemwood by pentylene glycol, as defined by a gas-liquid chromatography. The raw extract contained 87 -93 wt% pentylene glycol (solvent) and 6.5 -7.5 wt% polyphenolic compounds (mainly lignans) extracted from spruce. | |
|---|---|
| Hydroxymatairesinol | 70-80% |
| Secoisolariciresinol | 3-6% |
| Conidendrin | 4-7% |
| Lariciresinol | 1-3% |
| Liovile | 2-5% |
| Other lignans | 5-8% |

As is seen in Table 3B, spruce contains mainly lignans, of which the majority is hydroxymatairesinol. The other lignans mentioned in the Table are mainly oligolignans.

**Table 4A**

| | |
|---|---|
| Principal components of unrefined extraction solutions obtained from the bark of different wood species by hydrophilic extraction (Extractives in stemwood and knots of Acasia and Aspen trees, Suvi Pietarinen, Abo Akademi, Turku 2005). | |

| **Thuja occidentalis** | **Picea abies** |
|---|---|
| | |
| Sugars | Isorhapontine |
| Catechin | Astringin |
| Isorhapontine | Resveratrol-glycoside |
| Astringin | Tannins |
| Tannins | |
| | |

| **Pinus banksiana** | **Abies lasiocarpa** |
|---|---|
| Sugars | Sugars |
| Taxifolin | Resin acids |
| Isorhapontine | Tannins |
| Dihydromyrcetin | |
| Tannins | |
| | |

| **Betula pendula** | **Populus tremula** |
|---|---|
| Betuligenili glycoside | Undefined glycosides |
| Catechin | Tannins |
| Sugars | |
| Tannins | |
| | |

| **Pseudotsuga menziensii** | **Pinus mariana** |
|---|---|
| Sugars | Sugars |
| Taxifolin | Catechin |
| Catechin | Tannins |
| Tannins | |

**Table 4B**

| Principal components of unrefined extracts obtained from the knotty stemwood of a few pine and spruce species by hydrophilic extraction (Wilför et al., J. Agric. Food. Chem., 51, 26 (2003)), in percentages by weight of the total quantity of the components of the mixture extracted from the wood. | | |
|---|---|---|
| **Wood material** | **Compounds** | **wt%** |
| Picea abies | | |
| | **Lignans** | 53 |
| hydroxymatairesinol | | 41 |
| secoisolariciresinol | | 3 |
| α-conidendrin | | 7 |
| Oligolignans | | 12 |
| Abies sibirica | | |
| **Lignans** | | 33 |
| secoisolariciresinol | | 21 |
| lariciresinol | | 7 |
| Oligolignans | | 31 |
| **Juvabiones** | | 3 |
| | | |
| Abies balsamea | | |
| | **Lignans** | 22 |
| secoisolariciresinol | | 18 |
| lariciresinol | | 9 |
| | **Oligolignans** | 19 |
| | **Juvabiones** | 2 |
| | | |
| Pinus sibirica | | |
| | **Lignans** | 26 |
| lariciresinol | | 19 |
| isolariciresinol | | 3 |
| secolariciresinol | | 2 |
| Oligolignans | | 6 |
| | **Flavonoids** | 7 |
| pinosembrin | | 6 |
| | **Stilbenes** | 25 |
| dihydropinosylvin monomethyl ether | | 15 |
| pinosylvin | 3 | |
| dihydropinosylvin | | 2 |
| | | |
| Pinus contorta | | |
| | **Lignans** | 10 |
| nortrachelogenin | | 5 |
| liovile | | 3 |
| oligomers | **3** | |
| | **Flavonoids** | 20 |
| pinosembrin | | 15 |
| pinobanxin | **7** | |
| | **Stilbenes** | 15 |
| Pinosylvin monomethyl ether | | 9 |
| Pinosylvin | 6 | |

### Production of the compositions and their intermediate products

### Example 1

Example 1 describes an anti-micro-organistic composition (semi-finished product), in which there is used an unrefined solution obtained by hydrophilic extraction from Norway knotty spruce stemwood material.

### Intermediate product

| | |
|---|---|
| Ethylhexyl glycerine, anti-micro-organistic compound | 1 part |
| Raw extract obtained by pentylene glycol extraction from knotty stemwood chips of Picea abies (Norway spruce) | 6 parts |

The extract obtained from Norway spruce contained a mixture of phenolic compounds, where the composition of the phenolic compounds was in accordance with Table 3B. Ethylhexyl glycerine is efficient against Gram-negative bacteria, and it also has skin moisturizing and softening properties, wherefore it is used, among others, in deodorants for roughly 0.2 - 2 wt% solution of the total weight. Ethylhexyl glycerine is neither effective against Gram-positive bacteria, nor yeasts or fungi, and for this purpose there is therefore generally used another compound that is widely inhibiting for microbial growth, such as phenoxyethanol, which has a synergic positive effect with ethylhexyl glycerine. Phenoxyethanol is toxic, allergenic and skin irritative, and its highest acceptable limit of usage is roughly 1 wt% of the total weight of the composition.

An intermediate product according to example 1, composed of ethylhexyl glycerine and a widely anti-micro-organistic raw extract obtained in hydrophilic extraction from knotty stemwood material, which contains the compound mixture according to the invention, can be used in the same targets of usage as the known ethylhexyl glycerine and phenoxyethanol combination. Typical targets of usage are sun protection products, deodorants and cleaning cloths impregnated by the mixture.

### Example 2

### Composition

### Anti-scurf compositions

Compound mixtures according to the invention inhibit microbial growth on a wide scale, so that they can be used for replacing several specific preservatives in cosmetic compositions, i.e. specifically agents designed individually against Gram-negative and Gram-positive bacteria, fungi and yeast. Moreover, the cytotoxicity of said mixtures is low, and the compounds contained therein do not penetrate the skin, wherefore their skin irritation low, as opposite to certain conventionally used agents inhibiting the growth of anti-microbial agents on a wide scale, such as phenoxyethanol.

The mixtures according to the invention are effective against the yeast Malassezia furfur, wherefore they can be used as both wide-scale preservatives and also for inhibiting the action of the scurf forming yeast Malassezia furfur. Generally with scurf shampoo compositions it is necessary to separately add both preservatives, such as wide-scale bacteriostat phenoxyethanol, and further also separately an agent inhibiting the growth of scurf forming yeast. Owing to their toxicity and skin penetration, generally used wide-scale preservatives cannot be used in quantities sufficiently large in order to ensure the inhibition of the growth of the scurf-forming yeast; whereas the mixtures according to the invention have a low skin penetration, so that they can be used in relatively large quantities in cosmetic compositions.

### Example 2a

### Oil shampoo

| Ingredient | Wt%˙˙ |
|---|---|
| Sodium lauryl sulfate (Texapon N25) | 50 |
| PPG- 5 Laureth-5 (Aetoxal B) | 20 |
| Cocoamide DEA (Comperlan KD) | 3 |
| GranLux AOX-G3 Eco (10 wt% Spruce knot extract in propylen-1.3-diol) | 5 |
| Aqua and perfume | 22 |

Oil shampoo has a clear continuous water phase with 0.5 wt% mixture of compounds originating to spruce knots, extracted with alcohol (propyle-1.3-diol). Spruce knot extract gives anti-scurf properties (against M.furfur) to oil shampoo. The amount of water can be reduced substantially for example to 5 -20% if this oil shampoo is intended for large-scale consumers.

### Example 2b

### Combined hair conditioner and shampoo

| Ingredient | Wt%˙˙ |
|---|---|
| Coco-Betaine (Dehyton AB 30) | 20% |
| PEG-5 Cocoamide (Emulgin C4) | 20% |
| Cocoamid MEA (Comperlan KM) | 4% |
| Laurtrimonium Chloride (Dehyquat LT) | 4% |
| Granlux AOX-GL (10% Spruce knot extract in glycerine) | 4% |
| Aqua and perfume | 48% |

Combined hair conditioner and shampoo has also a clear continuous water phase as a carrier agent with 0.4 wt% mixture of compounds originating to spruce knots, extracted with alcohol (glycerine). Spruce knot extract gives anti-scurf properties (against M.furfur) to hair conditioner and shampoo. The amount of water can be reduced substantially for example to 5 -20% if this hair conditioner and shampoo is intended for large-scale consumers.

### Example 2c

| Vitaminized protein shampoo Ingredient | Wt%˙˙ |
|---|---|
| Polysorbate 20 (Tween 20) | 10 |
| Polysorbate 80 (Tween 80) | 5 |
| PEG-54 Hydrogenated Castor oil(Arlatone 289) | 10 |
| PPG-15 Stearyl Ether (Arlamol E) | 2 |
| Cocoamid DEA | 5 |
| Cocoamidopropyl Betain (Tegobetain L7) | 3 |
| Hydrolized collagen (Nutrilan L) | 5 |
| Vitamin F (Novarom) | 2.5 |
| Granlux AOX (10% Spruce knot extract in butylene glycol) | 5 |
| Aqua and perfume | 27.5 |

Vitaminized protein shampoo is a turbid O/W emulsion/dispersion with 0.5 wt% mixture of compounds originating to spruce knots, extracted with alcohol (butylene glycol). Spruce knot extract gives anti-scurf properties (against M.furfur) to hair conditioner and shampoo. The amount of water can be reduced substantially for example to 5 -20% if this vitaminized shampoo is intended for large-scale consumers. This composition also contains an amphoteric surfactant (cocoamidopropyl betain), foaming agent (Cocoamid DEA), and ionic surfactants (polysorbates).

### Appendix 1

### Figure 1

Figure 1 illustrates different phenolic compounds that can be obtained by a hydrophilic extraction of wood material originated to knotty stemwood or bark of pine.

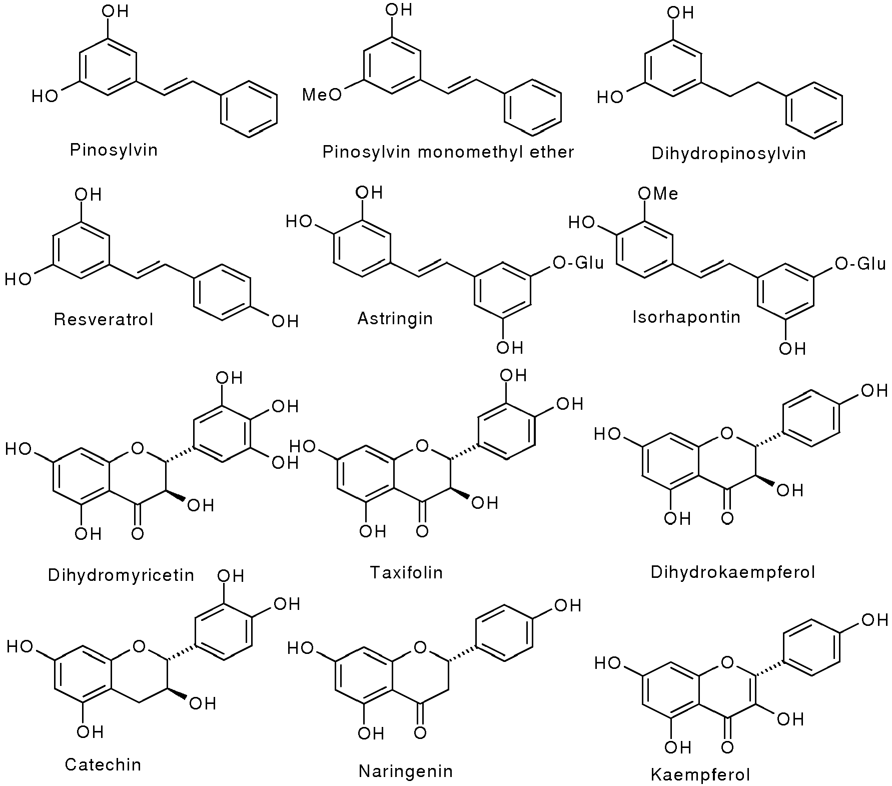

### Appendix 2

### Figure 1 continued

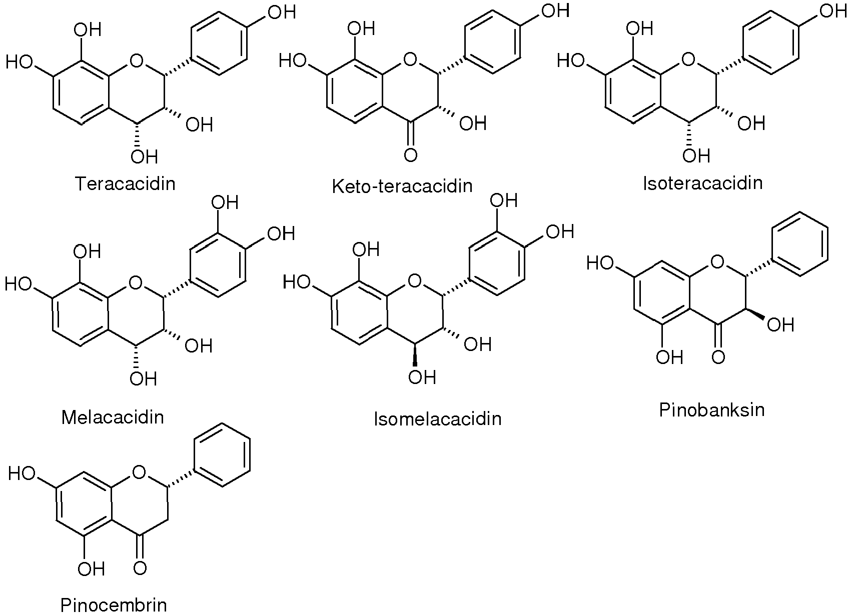

### Appendix 3

### Figure 2

Figure 2 illustrates the different phenolic and polyphenolic compounds contained in the bark of betula pendula (birch tree).

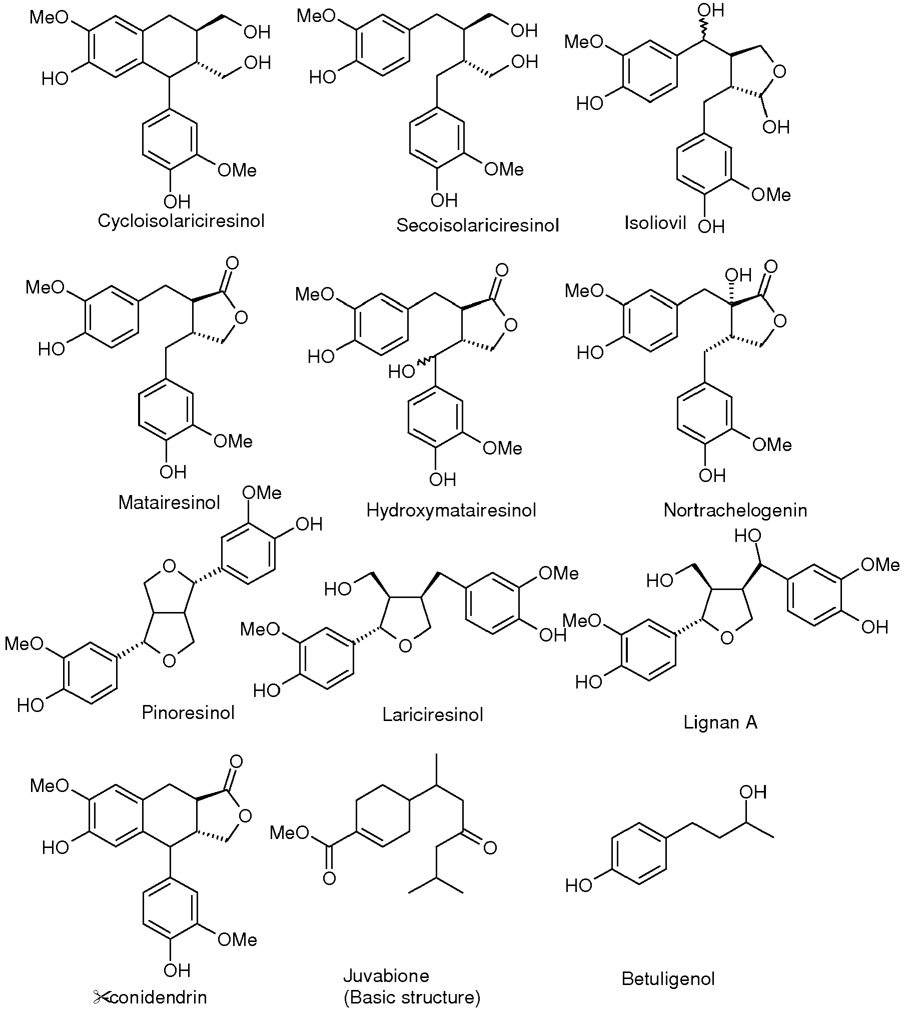

### Appendix 4

**Table 5A**

| | |
|---|---|
| Antioxidative capacity of the lipids in polyphenols-bearing unrefined extracts obtained in hydrophilic extraction from knotty stemwood or wood bark, as expressed in terms of extract concentration EC50 µg/L, which inhibits 50% of the peroxidation of lipids. | |
| knotty stemwood | EC50 µg/L |
| obtained extracts | |
| | |
| Acacia crassicarpa | 19 |
| Abies pectinata | 21 |
| Picea glauca | 24 |
| Acasia mangium | 24 |
| Tsuga canadensis | 27 |
| Picea sitchensis | 28 |
| Tsuga heterophylla | 28 |
| Eukalyptus globukus | 57 |
| Abies lasiocarpa | 59 |
| Populus gradidentata | 61 |
| Pinus resinosa | 61 |
| Fagus sylvatica | 91 |
| Populus tremuloidis | 135 |
| Pinus strobes | 159 |
| Populus tremula | 317 |
| | |
| extracts obtained from wood bark | EC50 µg/L |
| | |
| Picea abies | 49 |
| Betula pendula | 81 |
| Pycnogenol | 84 |
| Pseudotsugamenziensii | 84 |
| Thuja occidentalis | 131 |
| Pinus banksiana | 143 |
| Populus tremula | 213 |
| Abies lasiocarpa | 316 |

### Appendix 5

**Table 5B**

| | |
|---|---|
| Comparison of the antioxidative capacity of the lipids in pure polyphenol extracts isolated from certain wood materials, as expressed by the extract concentration EC50 µg/L, which inhibits 50 % of the peroxidation of the lipids. | |

| Compound | EC50 µg/L |
|---|---|
| Cyclolariciresinol | 17 |
| Pinoresinol | 20 |
| Melacasidine | 36 |
| Secoisolariciresinol¹ | 37 |
| Taxifolin¹ | 46 |
| Pinosylvin | 50 |
| Teracasidine | 50 |
| Nortrahelogeniini¹ | 53 |
| Hydroxymatairesinol¹ | 58 |
| Matairesinol ¹ | 99 |
| Lariciresinol¹ | 126 |
| Dihydrokaempferol | 488 |
| Pinosembrin | 1135 |

| | |
|---|---|
| 1: Willför et al,. J. Agric. Food. Chem, 51. 26 (2003). | |

Tables 5C and 5D further represent the free radicals capturing capacity of certain pure polyphenols and unrefined polyphenol extracts with respect to peroxide radicals.

### Appendix 6

**Table 5C.**

| | |
|---|---|
| Peroxide radicals /(mmol) capturing capacity of unrefined polyphenols-bearing extracts obtained in hydrophilic extraction from knotty stemwood or wood bark, as expressed in capacity per gram of extract. | |

| extracts obtained from knotty stemwood | Wood material, capturing capacity mmol/g |
|---|---|
| Acacia crassicarpa | 21 |
| Eukalyptus globulus | 7.8 |
| Picea glauca | 7.8 |
| Abies pectinata | 6.8 |
| Tsuga canadensis | 6.8 |
| Acasia mangium | 6.8 |
| Tsuga heterophylla | 5.8 |
| Larix lariciana | 5.8 |
| Larix sibrica | 5.8 |
| Picea mariana | 5.8 |
| Picea sitchensis | 4.9 |
| Pinus sylvestris | 4.9 |
| Thuja plicata | 3.9 |
| Populus gradidentata | 3.9 |
| Fagus sylvatica | 2.9 |
| Abies lasiocarpa | 2.7 |
| Pinus resinosa | 2.7 |
| Pinus banksiana | 1.9 |
| Pinus strobus | 1.1 |
| Populus tremuloidis | 0.39 |
| Populus tremula | 0.29 |
| | |
| wood bark obtained extracts | mmol/g |
| | |
| Pseudotsuga menziensii | 4.9 |
| Pycnogenol | 4.9 |
| Pinus banksiana | 3.1 |
| Betula pendula | 2.9 |
| Picea abies | 2.9 |
| Thuja occidentalis | 1.9 |
| Abies lasiocarpa | 0.58 |
| Populus tremula | 0.29 |

### Appendix 7

**Table 5D**

| Comparison of the peroxide radicals (mmol) capturing capacity of certain pure polyphenol extracts isolated from wood material, as expressed per gram of extract. | |
|---|---|
| Compound | capturing capacity mmol/g |
| Melacasidine | 20 |
| Taxifolin¹ | 16 |
| Cyclolariciresinol | 12 |
| Secoisolariciresinol¹ | 8.5 |
| Pinoresinol | 7.8 |
| Tetra casidin | 7.8 |
| Nortrachelogenin¹ | 5.9 |
| Hydroxymatairesinol¹ | 5.6 |
| Matairesinol¹ | 2.9 |
| Lariciresinol¹ | 2.7 |
| Dihydrokaempferol | 0.78 |
| Pinosylvin | 0.78 |
| Pinosembrin | 0.49 |

| | |
|---|---|
| 1: Willför et al,. J. Agric. Food. Chem., 51, 7600-7606 (2003). | |

## Claims

1. A method comprising following steps:
- in the composition, there is included an aqueous carrier agent with either hydrophobic or hydrophilic nature, selected according to the target of usage and surface active agents, conventionally used in a cosmetic composition, perfumes and possible dispersing agents, and
- in connection with producing the carrier, there is included a widely anti-microbial compound mixture for the amount of 0.1- 5 wt% of the total weight of said composition, which compound mixture is obtained by pulverizing wood material and/or by extracting possibly pulverized wood material, so that said compound mixture contains at least two different compounds selected from among the following group: lignans according to formula IA 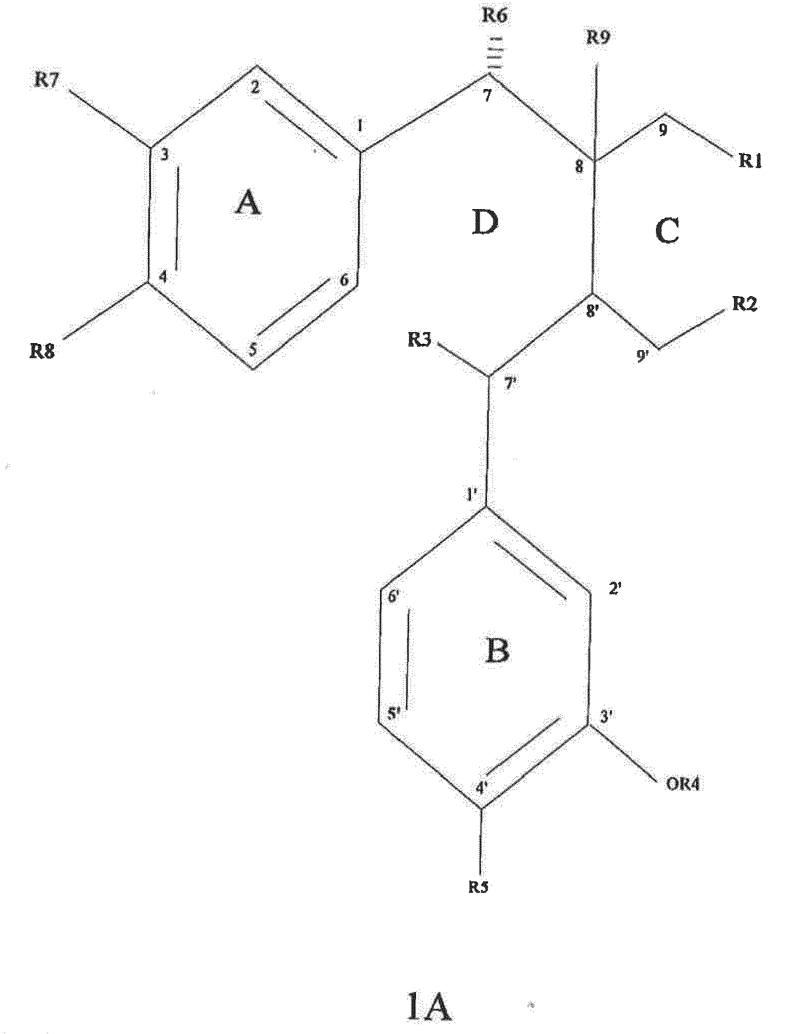 wherein
R1 or R2 denote, irrespective of each other, residue selected from the groups hydrogen, OH or =O,
or either one of the residues R1 or R2 denotes the oxygen atom -O- bound to carbons 9 and 9', and now forms with carbons 8, 8', 9, 9' a 5-membered oxygenous heterocyclic ring C,
R3 denotes hydrogen or residue selected from the group OH, =O, or it forms a bond to the carbon 6, so that the carbons 6, 1, 7, 8, 8', 7' form a cyclohexane ring that is condensed with the phenyl ring A, and possibly also a ring with C,
R4 denotes hydrogen or methyl,
R5 denotes hydrogen or residue selected from the groups OH and OCH₃,
R6 denotes hydrogen or hydroxy,
R7 and R8 denote, irrespective of each other, hydrogen or residue selected from the groups OH and OCH₃.
R9 denotes H or in case of nortrachelogenin R9 denotes OH
or lignans of general formula IB 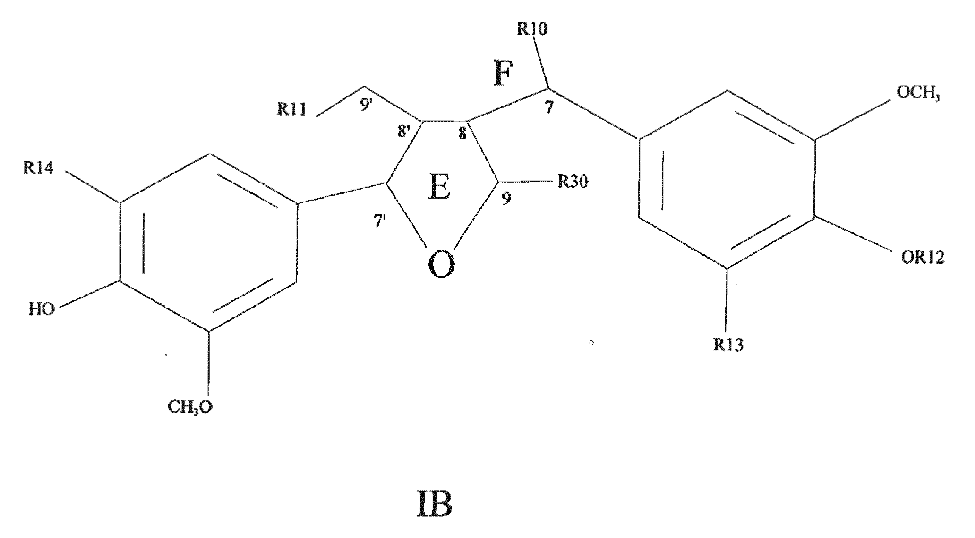 wherein
R10 denotes hydrogen or hydroxy
R11 denotes hydroxy or oxygen, which is bound by a bond to the carbons 7 and 9', forming now an oxygenous non-aromatic 5-membered heterocyclic ring (tetrahydrofuran) F with the carbons 7, 8, 8', 9', which ring is condensed in the hetero ring E (tetrahydrofuran) at the carbons 8, 8',
R12 denotes hydrogen or methyl,
R13 denotes hydrogen or methoxy,
R14 denotes hydrogen or methoxy,
R30 denotes hydrogen or group =O and
stilbenes according to general formula II 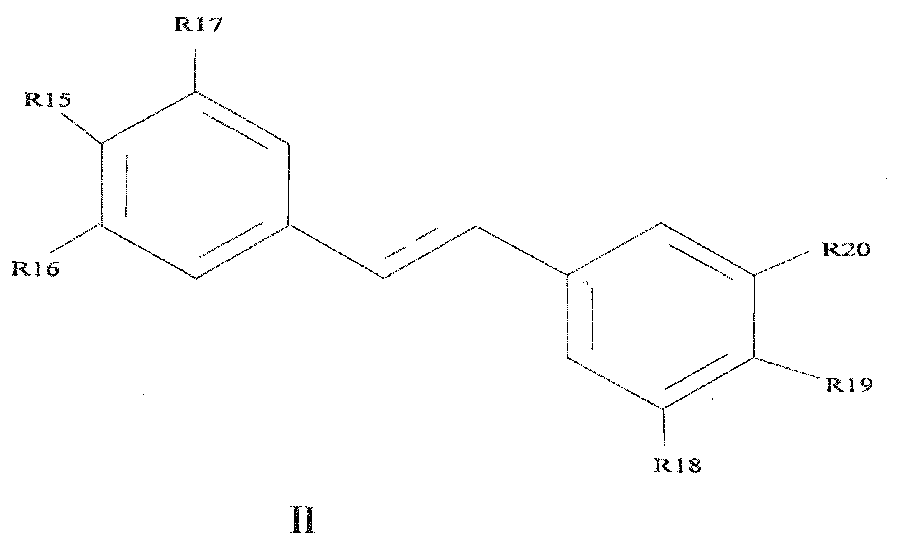 wherein
R15 denotes hydrogen or hydroxy
R16 denotes residue selected from the groups H, OH, OCH₃,
R17 denotes residue selected from the groups OH or OCH₃,
R18 and R19 denote, irrespective of each other, hydrogen or hydroxy,
R20 denotes residue selected from the groups hydrogen, OGlu and juvabiones according to general formula III, 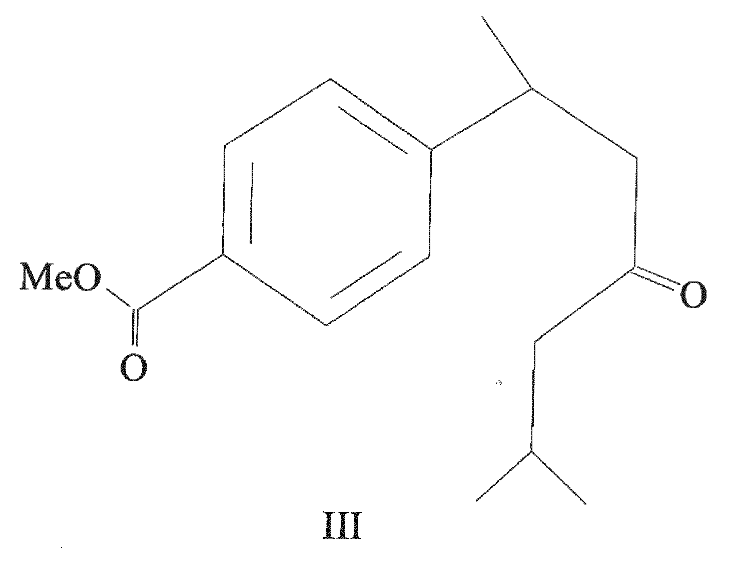 and flavonoids according to general formula IV 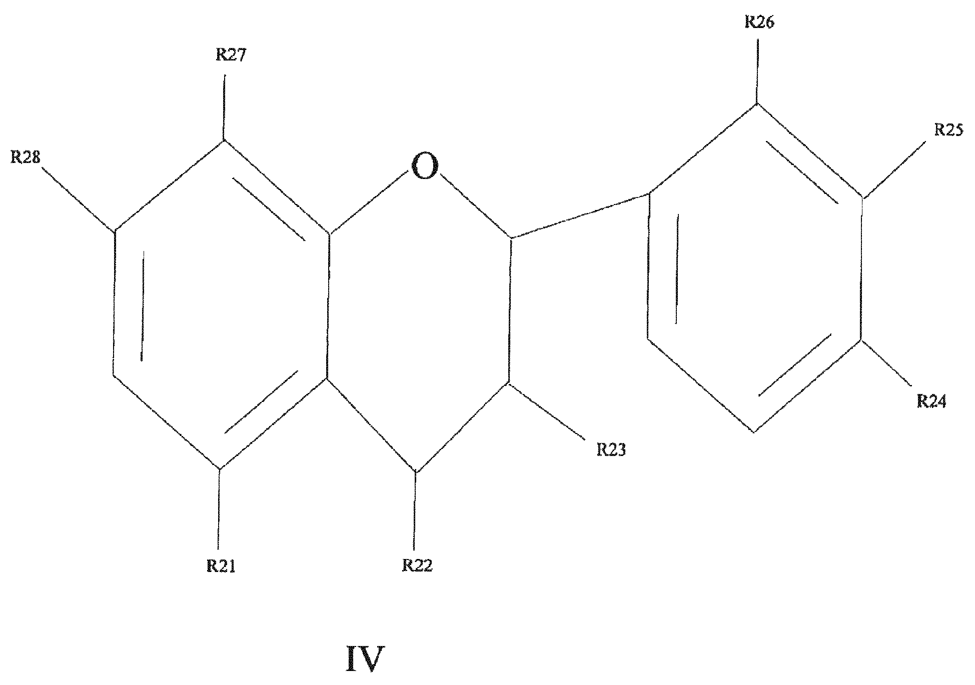 wherein
R21 denotes residue selected from the groups H, OH,
R22 denotes residue selected from the groups H, OH, =O,
R23 denotes residue selected from the groups H, OH
R24, R25, R26 denote, irrespective of each other, hydrogen or hydroxy,
R28 and R27 denote, irrespective of each other, hydrogen or hydroxy,and betulin, betulonic acid, betulinic acid, betuloinic acid as well as their ester or ether derivatives, as well as stereoisomers; said mixture further containing oligomers of said compounds, providing, however, that the mixture includes lignans, particularly 7-hydroxymatairesinol or secoisolariciresinol 50 - 99.9 wt%, stilbenes, particularly pinosylvin or its ester or ether derivatives 0 - 70 wt%, oligomers of lignans, stilbenes, juvabiones or flavonoids 1 - 31 wt%, further providing that the mixture contains at least one compound selected from the following group: 7-hydroxymatairesinol, conidendrin, conidendric acid, alpha-conidendrin, alpha-conidendric acid, isohydroxymatairesinol, cyclolariciresinol, secoisolariciresinol, anhydrosecoisolariciresinol and stilbenes as well as their ester or ether derivatives and stereoisomers, wherein in said method the content of the said compound mixture is kept in such that with said content,
- the compounds contained in the compound mixture do not irritate the skin in a so-called single patch test, and
- the compounds contained in the compound mixture are such that the cytotoxicity of said compounds, as measured in ethanol for a HaCat cell culture after 24 incubations, is lower than the cytotoxicity of 0.02 - 0.1 wt% BHT dissolved in ethanol in the same incubation conditions, preferably lower than the cytotoxicity of 0.01 - 0.05 wt% BHT dissolved in ethanol in the same incubation conditions and
- the compound mixture also has in the composition a wide-scale anti-microbial effect against Gram- bacteria, Gram+ bacteria, yeast and fungi wherein said wide scale anti-microbial effect means that the compound mixture has effects for inhibiting, from among Gram-negative bacteria, at least the growth of E. coli, Ps. aeruginosa, Ps. putida, KI. pneimoniae; from among Gram-positive bacteria at least the growth of S. aureus; from among yeasts at least the growth of scurf-causing yeast, M. furfur and C. albicans; and from among fungi at least the growth of A. niger;
for production of a hair care product, including an aqueous carrier agent; a widely anti-microbial compound mixture mentioned above, that inhibits the growth of scurf-causing yeast, Malassezia furfur; surface active agents, perfumes and possible dispersing agents.

2. The use of the method according to claim 1 for production of a hair care product defined in claim 1 which additionally contains a narrow-scale anti-microbial agent.

3. The use of the method according to claim 1 or 2 for production of a hair care product, defined in claim 1, wherein in the compound mixture the oligomers are oligolignans.

4. The use of the method according to any of the preceding claims for production of a hair care product, defined in claim 1, wherein the method comprises obtaining the compound mixture by pulverizing wood material from at least two different wood species and/or by extracting possibly pulverized wood material from at least two different wood species, so that the wood material is preferably wood knot material or stemwood material from adjacent to knots, and that in the extraction process, there is advantageously employed an organic hydrophilic solvent, preferably a hydrocarbon-carbonyl-based organic solvent especially a solution containing alkyl carbonyls such as alcohols or ketones.

5. The use of the method according to claim 4 for production of a hair care product, defined in claim 1, wherein in the method the compound mixture is obtained by extracting the wood material of pine and spruce, and that the compound mixture contains both stilbenes that lignans, 50 - 99.9% of the lignans being obtained from spruce knot material or spruce stemwood material adjacent to knots, and 0.1 -50% of the lignans is obtained from pine knot material or pine stemwood material adjacent to knots.

6. The use of the method according to any of the preceding claims for production of a hair care product, defined in claim 1, wherein the method comprises including a narrow-scale anti-microbial agent which is preferably betulin, betulonic acid, betulinic acid, betuloinic acid or their derivatives obtained from birch bark, or resveratrol or its derivative obtained from spruce bark, or ethylhexyl glyceryl.

7. The use of the method according to any of the preceding claims 1-6 for production of a hair care product, defined in claim 1, wherein the method comprises including as additives, antioxidative agents such as natural or synthetic vitamins, for example vitamin A, B, C, D, E, provitamin B5, vitamin B3, L-ascorbic acid, vitamin E, antioxidants obtained from plants, such as antioxidants contained in green tea, antioxidants contained in flaxseed, antioxidants contained in horse chestnut, beta carotenes, selenium, glutamine, ubiquinone, glycolic acid, growth hormones and kinetin.

8. The use of the method according to any of the preceding claims for production of a hair care product, defined in claim 1, wherein the method comprises including the compound mixture that contains at least two different compounds, selected from the following group:
Lignans:
- matairesinol, hydroxymatairesinol, oxomatairesinol, didemethyl matairesinol, didemethyl matairesinol, isohydroxymatairesinol, epi-isohydroxymatairesinol and their stereoisomers, among which let us particularly point out hydroxymatairesinol stereoisomers 7S, 8R; 8'R-hydroxymatairesinol and 7R, 8R, 8'R-allohydroxymatairesinol, and their stereoisomers and ester or ether derivatives,
- secoisolariciresinol, isolariciresinol, lariciresinol, pinoresinol, dimethyl secoisolariciresinol, 7-hydroxysecoisolariciresinol, cyclolariciresinol, cycloisolariciresinol and their stereoisomers and ester or ether derivatives,
- nortrachelogenin and its stereoisomers and ester or ether derivatives
- enterolactone and its stereoisomers and ester or ether derivatives,
- conidendrin, α-conidendrin and their stereoisomers and ester or ether derivatives,
- lignan A and its stereoisomers and ester or ether derivatives,
- liovile and its stereoisomers and ester or ether derivatives;
Juvabiones:
- juvabiones and their stereoisomers and ester or ether derivatives;
Stilbenes:
- pinosylvin, dihydropinosylvin, pinosylvin monomethyl ether, dihydropinosylvin monomethyl ether, resveratrol, astringin, isorhapontine, and their stereoisomers and ester or ether derivatives,
Flavonoids:
- pinosembrin, catechin, pinobanxin, kaempferol, dihydrokaempferol, taxifolin, naringenin, teracasidine, ketoteracasidine, isoteracasidine, melacasidine, isomelacasidine and their stereoisomers and ester or ether derivatives,
- betulin, betulonic acid, betulinic acid, betuloinic acid or their ester derivative,
as well as the glycosidized forms of these compounds and their oligomers, such as trimers and tetramers.

9. The use of the method according to claim 1 for production of a hair care product, defined in claim 1, wherein the method comprises extracting the wood material with an organic lipophilic solvent, such as hydrocarbon, as well as with an organic hydrophilic solvent, such as an organic lower alkyl carbonyl compound, and that the lipophilic and/or hydrophilic extraction solution forms part of the composition carrier agent.

10. The use of the method according to claim 1 for production of a hair care product, defined in claim 1, wherein the method comprises extracting the wood material with a monovalent or multivalent alcohol or their mixture wherein the extraction solution is preferably the multivalent lower alkyl alcohol which is advantageously selected from the following groups: lower alkyl diode, such as propyl glycol, butyl glycol, pentyl glycol, octyl glycol, lower alkyl triol, and their esters.

11. The use of the method according to claim 10 for production of a hair care product, defined in claim 1, wherein the method comprises employing the solution that is a lower alkane, preferably hexane or heptane as the wood material extraction solution.

12. The use of the method according to claim 9 for production of a hair care product, defined in claim 1, wherein in the method the extraction solution and the carrier present in the composition contains a hydrocarbon-based solution, or a ketone, or an inorganic anion-cation solution or an ionogenic solution, or an ester of a trivalent alcohol.

## Patentansprüche

1. Verfahren, das folgende Schritte umfasst:
- in der Zusammensetzung sind ein wässriges Trägermittel mit entweder einer hydrophoben oder einer hydrophilen Natur, das gemäß dem Ziel des Gebrauchs ausgewählt wird, und grenzflächenaktive Stoffe, die herkömmlicherweise in einer kosmetischen Zusammensetzung, in Parfüms und möglicherweise in dispergierenden Mitteln verwendet werden, enthalten; und
- in Verbindung mit dem Herstellen des Trägers ist eine breite antimokrobielle Verbindungsmischung enthalten, die eine Menge von 0,1 bis 5 Gew.% des Gesamtgewichts der Zusammensetzung ausmacht, wobei die Verbindungsmischung erhalten wird, indem Holzmaterial pulverisiert wird und/oder indem das möglicherweise pulverisierte Holzmaterial extrahiert wird, so dass die Verbindungsmischung mindestens zwei unterschiedliche Verbindungen enthält, die aus der folgenden Gruppe ausgewählt sind: Lignane gemäß der allgemeinen Formel IA: 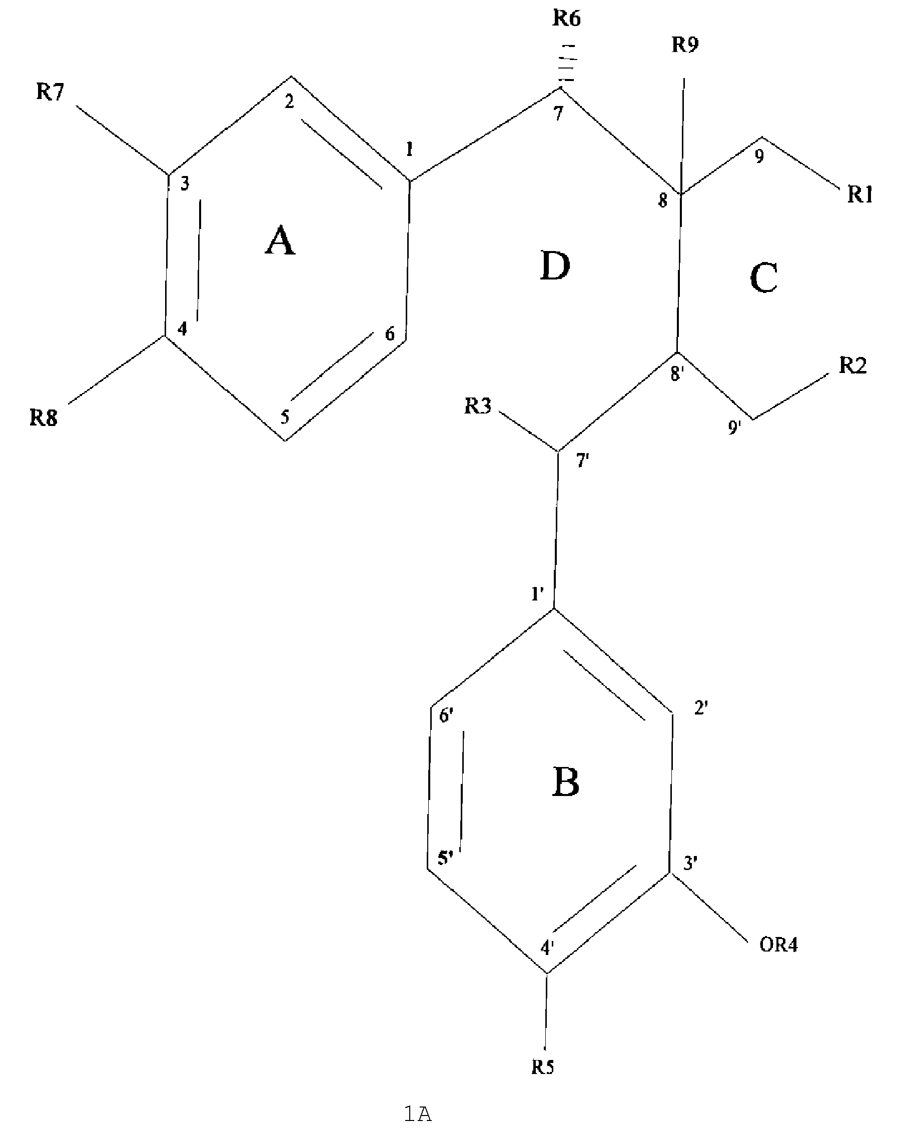 wobei
R1 oder R2 unabhängig voneinander einen Rest bezeichnen, der ausgewählt ist aus den Gruppen Wasserstoff, OH oder =0,
oder einer der Reste R1 oder R2 das Sauerstoffatom -O-bezeichnet, das an den Kohlenstoff an der Stelle 9 und 9' gebunden ist, und jetzt mit den Kohlenstoffatomen 8, 8', 9, 9' einen 5-gliedrigen, sauerstoffhaltigen heterozyklischen Ring C bildet,
R3 Wasserstoff oder einen Rest bezeichnet, der ausgewählt ist aus der Gruppe OH, =0, oder eine Bindung an den Kohlenstoff 6 bildet, so dass die Kohlenstoffatome 6, 1, 7, 8, 8', 7' einen Cyclohexanring, der mit dem Phenylring A kondensiert ist, und möglicherweise auch einen Ring mit C bilden,
R4 Wasserstoff oder Methyl bezeichnet,
R5 Wasserstoff oder einen Rest bezeichnet, der ausgewählt ist aus den Gruppen OH und OCH₃,
R6 Wasserstoff oder Hydroxy bezeichnet,
R7 und R8 unabhängig voneinander Wasserstoff oder einen Rest bezeichnen, der ausgewählt ist aus den Gruppen OH und OCH₃,
R9 H bezeichnet oder wobei R9 im Falle von Nortrachelogenin OH oder Lignane von der folgenden allgemeinen Formel IB bezeichnet: 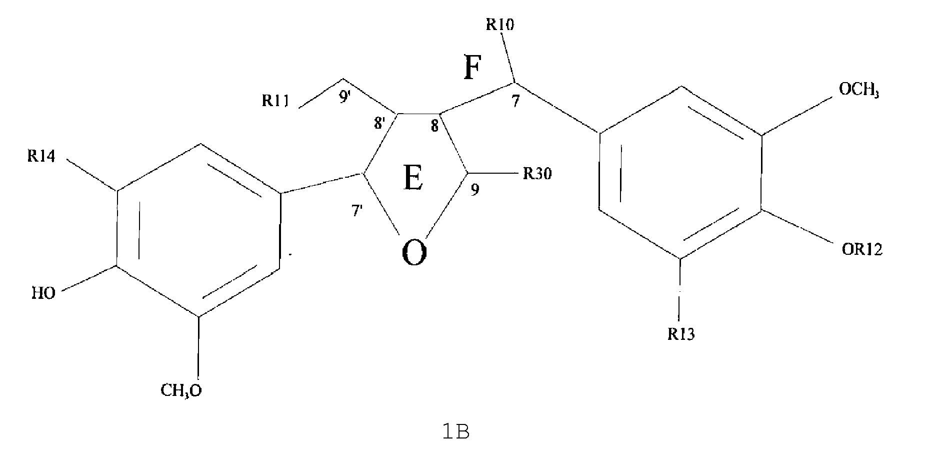 wobei
R10 Wasserstoff oder Hydroxy bezeichnet,
R11 Wasserstoff oder Sauerstoff bezeichnet, der durch eine Bindung an die Kohlenstoffatome an der Stelle 7 und 9' gebunden ist, die jetzt einen sauerstoffhaltigen, nicht-aromatischen, 5-gliedrigen heterozyklischen Ring (Tetrahydrofuran) F mit den Kohlenstoffatomen 7, 8, 8', 9' bilden, wobei der Ring in dem Heteroring E (Tetrahydrofuran) an den Kohlenstoffatomen 8, 8' kondensiert ist,
R12 Wasserstoff oder Methyl bezeichnet,
R13 Wasserstoff oder Methoxy bezeichnet,
R14 Wasserstoff oder Methoxy bezeichnet,
R30 Wasserstoff oder eine Gruppe =0 bezeichnet und
Stilbene gemäß der folgenden allgemeinen Formel II: 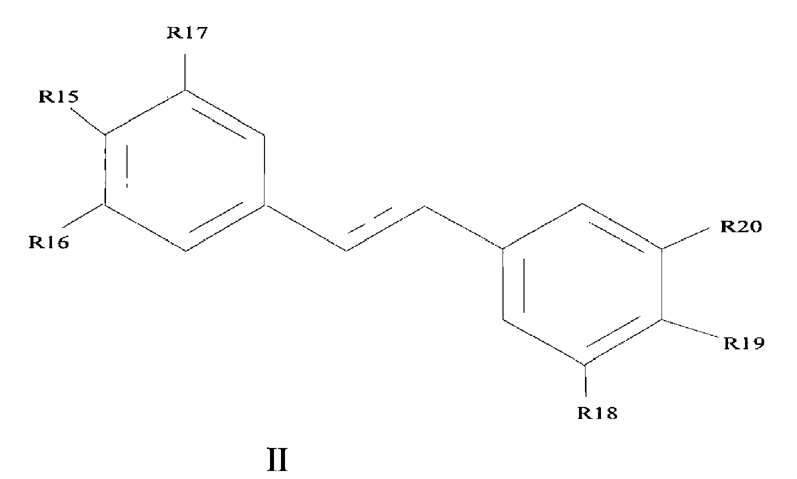 wobei
R15 Wasserstoff oder Hydroxy bezeichnet,
R16 einen Rest bezeichnet, der ausgewählt ist aus den Gruppen H, OH, OCH₃,
R17 einen Rest bezeichnet, der ausgewählt ist aus den Gruppen OH oder OCH₃,
R18 und R19 unabhängig voneinander Wasserstoff oder Hydroxy bezeichnen,
R20 einen Rest bezeichnet, der ausgewählt ist aus den Gruppen Wasserstoff, OGlu und Juvabione gemäß der folgenden allgemeinen Formel III: 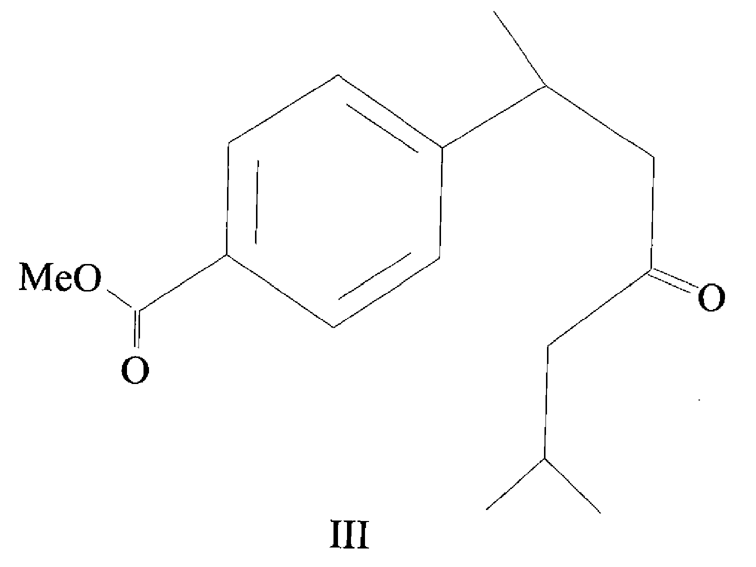 und aus Flavonoiden gemäß der folgenden allgemeinen Formel IV: 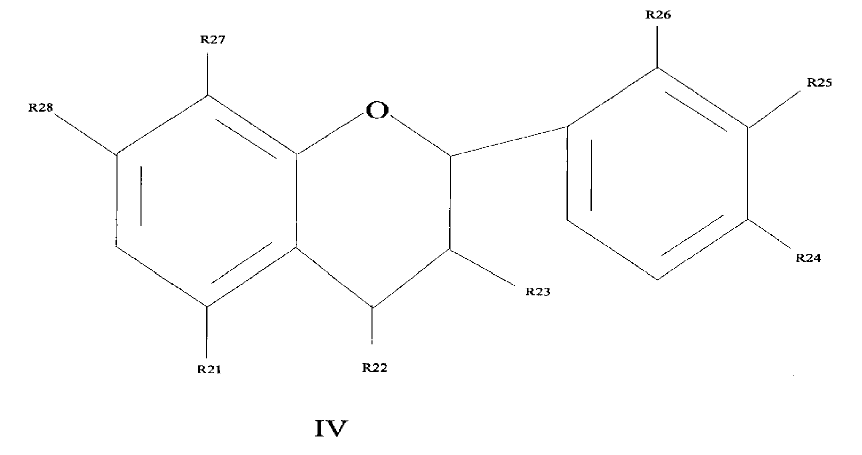 wobei
R21 einen Rest bezeichnet, der ausgewählt ist aus den Gruppen H, OH,
R22 einen Rest bezeichnet, der ausgewählt ist aus den Gruppen H, OH, =0,
R23 einen Rest bezeichnet, der ausgewählt ist aus den Gruppen H, OH,
R24, R25, R26 unabhängig voneinander Wasserstoff oder Hydroxy bezeichnen,
R28 und R27 unabhängig voneinander Wasserstoff oder Hydroxy bezeichnen, und Betulin, Betulonsäure, Betulinsäure, Betuloinsäure sowie deren Ester- oder Etherderivate sowie deren Stereoisomere; wobei die Verbindungsmischung ferner Oligomere dieser Verbindungen enthält, vorausgesetzt jedoch, dass die Mischung Lignane enthält, insbesondere 7-Hydroxymatairesinol oder Secoisolariciresinol von 50 bis 99,9 Gew.%, Stilbene, insbesondere Pinosylvin oder seine Ester- oder Etherderivate für 0 bis 70 Gew.%, Oligomere von Lignanen, von Stilbenen, von Juvabionen oder von Flavonoiden von 1 bis 31 Gew.%, ferner vorausgesetzt, dass die Mischung mindestens eine Verbindung enthält, die aus der folgenden Gruppe ausgewählt ist: 7-Hydroxymatairesinol, Conidendrin, Conidendrinsäure, alpha-Conidendrin, alpha-Conidendrinsäure, Isohydroxymatairesinol, Cyclolariciresinol, Secoisolariciresinol, Anhydrosecoisolariciresinol und Stilbene ebenso wie deren Ester- oder Etherderivate und Stereoisomere, wobei in dem Verfahren der Inhalt der Verbindungsmischung derart gehalten wird, dass mit dem Inhalt
- die Verbindungen, die in der Verbindungsmischung enthalten sind, die Haut in einer so genannten einzelnen Verträglichkeitsprüfung mit einem Testpflaster (Patchtest) nicht reizen, und
- die Verbindungen, die in der Verbindungsmischung enthalten sind, derart sind, dass die Cytotoxizität der Verbindungen, wenn sie in Ethanol für eine HaCat-Zellkultur nach 24 Inkubationen gemessen wird, niedriger als die Cytotoxizität von 0,02 bis 0,1 Gew.% BHT ist, die in Ethanol unter denselben Inkubationsbedingungen aufgelöst ist, bevorzugt niedriger als die Cytotoxizität von 0,01 bis 0,05 Gew.% BHT, die in Ethanol unter denselben Inkubationsbedingungen aufgelöst ist, und
- die Verbindungsmischung in der Zusammensetzung auch im breiten Maßstab eine antimikrobielle Wirkung gegen gramnegative Bakterien, grampositive Bakterien, Hefe und Pilze aufweist, wobei die antimikrobielle Wirkung im breiten Maßstab bedeutet, dass die Verbindungsmischung Wirkungen aufweist, um unter den gramnegativen Bakterien mindestens das Wachstum von E. coli, Ps. aeruginosa, Ps. putida, Kl. pneimoniae zu hemmen; unter den grampositiven Bakterien mindestens das Wachstum von S. aureus; unter den Hefen mindestens das Wachstum von Schuppen verursachenden Hefen, M furfur und C. albicans; und unter den Pilzen mindestens das Wachstum von A. niger;
für eine Herstellung eines Haarpflegeprodukts, das ein wässriges Trägermittel enthält; eine breite antimikrobielle Verbindungsmischung, die oben erwähnt ist und die das Wachstum von einer Schuppen verursachende Hefe, Malassezia furfur, hemmt; grenzflächenaktive Stoffe, Parfüms und mögliche dispergierende Mittel.

2. Verwendung des Verfahrens nach Anspruch 1 für eine Herstellung eines Haarpflegeprodukts, das in Anspruch 1 definiert ist, das zusätzlich ein im beschränkten Maße antimikrobielles Mittel enthält.

3. Verwendung des Verfahrens nach Anspruch 1 oder 2 für eine Herstellung eines Haarpflegeprodukts, das in Anspruch 1 definiert ist, wobei in der Verbindungsmischung die Oligomere Oligolignane sind.

4. Verwendung des Verfahrens nach einem der vorhergehenden Ansprüche für eine Herstellung eines Haarpflegeprodukts, das in Anspruch 1 definiert ist, wobei das Verfahren umfasst, dass die Verbindungsmischung erhalten wird, indem Holzmaterial aus mindestens zwei unterschiedlichen Holzarten pulverisiert wird und/oder indem möglicherweise pulverisiertes Holzmaterial aus mindestens zwei unterschiedlichen Holzarten extrahiert wird, derart, dass das Holzmaterial bevorzugt Holzastmaterial oder Stammholzmaterial aus der Nachbarschaft zu Ästen ist, und dass in dem Extraktionsprozess vorteilhaft ein organisches hydrophiles Lösemittel, vorzugsweise ein auf Kohlenwasserstoff-Carbonyl basierendes organisches Lösemittel angewendet wird, insbesondere eine Lösung, die Alkylcarbonyle wie etwa Alkohole oder Ketone enthält.

5. Verwendung des Verfahrens nach Anspruch 4 für eine Herstellung eines Haarpflegeprodukts, das in Anspruch 1 definiert ist, wobei in dem Verfahren die Verbindungsmischung durch ein Extrahieren des Holzmaterials von einer Kiefer und von einer Fichte erhalten wird, und dass die Verbindungsmischung sowohl Stilbene als auch Lignane enthält, wobei 50 bis 99,9 % der Lignane aus einem Holzmaterial von Fichtenholzästen oder aus einem Fichtenstammholzmaterial, das benachbart zu Ästen liegt, erhalten werden, und wobei 0,1 bis 50 % der Lignane aus einem Holzmaterial von Kiefernholzästen oder aus einem Kiefernstammholzmaterial, das benachbart zu Ästen liegt, erhalten werden.

6. Verwendung des Verfahrens nach einem der vorhergehenden Ansprüche für eine Herstellung eines Haarpflegeprodukts, das in Anspruch 1 definiert ist, wobei das Verfahren umfasst, dass es ein im beschränkten Maße antimikrobielles Mittel enthält, das vorzugsweise Betulin, Betulonsäure, Betulinsäure, Betuloinsäure oder deren Derivate, die von der Birkenrinde gewonnen werden, oder Resveratrol oder deren Derivate, die von der Fichtenrinde gewonnen werden, oder Ethylhexylglyceryl enthält.

7. Verwendung des Verfahrens nach einem der vorhergehenden Ansprüche 1 bis 6 für eine Herstellung eines Haarpflegeprodukts, das in Anspruch 1 definiert ist, wobei das Verfahren umfasst, dass es Zusatzstoffe, antioxidative Mittel wie etwa natürliche oder synthetische Vitamine enthält, zum Beispiel Vitamin A, B, C, D, E, Provitamin B5, Vitamin B3, L-Ascorbinsäure, Vitamin E, Antioxidanzien, die aus Pflanzen erhalten werden, zum Beispiel Antioxidanzien, die in dem grünen Tee enthalten sind, Antioxidanzien, die im Leinsamen enthalten sind, Antioxidanzien, die im Leinsamen enthalten sind, Antioxidanzien, die in der Rosskastanie, in Betacarotin, Selen, in Glutuamin, in Ubichinon, in Glykolsäure, in Wachstumshormonen und in Kinetin enthalten sind.

8. Verwendung des Verfahrens nach einem der vorhergehenden Ansprüche für eine Herstellung eines Haarpflegeprodukts, das in Anspruch 1 definiert ist, wobei das Verfahren umfasst, dass die Verbindungsmischung mindestens zwei verschiedene Verbindungen enthält, die aus der folgenden Gruppe ausgewählt sind:
Lignane:
- Matairesinol, Hydroxymatairesinol, Oxomatairesinol, Didemethyl-matairesinol, Isohydroxymatairesinol, Epiisohydroxymatairesinol und ihre Stereoisomere, unter denen besonders die Hydroxymatairesinol-Stereoisomere 7S, 8R; 8'R-Hydroxymatairesinol und 7R, 8R, 8'R-Allohydroxymatairesinol und ihre Stereoisomere und Ester- oder Etherderivate hervorzuheben sind,
- Secoisolariciresinol, Isolariciresinol, Lariciresinol, Pinoresinol, Dimethylsecoisolariciresinol, 7-Hydroxysecoisolariciresinol, Cycloisolariciresinol und ihre Stereoisomere und Ester- oder Etherderivate,
- Nortrachelogenin und seine Stereoisomere und Ester- oder Etherderivate,
- Enterolacton und seine Stereoisomere und Ester- oder Etherderivate,
- Conidendrin, α-Conidendrin und ihre Ester- oder Etherderivate,
- Lignan A und seine Stereoisomere und Ester- oder Etherderivate,
- Liovil und seine Stereoisomere und Ester- oder Etherderivate;
Juvabione:
- Juvabione und deren Stereoisomere und Ester- oder Etherderivate;
Stilbene
- Pinosylvin, Dihydropinosylvin, Pinosylvinmonomethylether, Dihydropinosylvinmonomethylether, Resveratrol, Astringin, Isorhapontin und deren Stereoisomere und Ester- oder Etherderivate,
Flavonoide:
- Pionosembrin; Catechin, Pinobanxin, Kaempferol, Dihydrokaempferol, Taxifolin, Naringenin, Teracasidin, Ketoteracasidin, Isoteracasidin, Melacasidin, Isomelacasidin und deren Stereoisomere und Ester- oder Etherderivate,
- Betulin, Betulonsäure, Betulinsäure, Betuloinsäure oder deren Esterderivate,
sowie die glycosidierten Formen von diesen Verbindungen und deren Oligomere wie etwa Trimere und Teramere.

9. Verwendung des Verfahrens nach Anspruch 1 für eine Herstellung eines Haarpflegeprodukts, das in Anspruch 1 definiert ist, wobei das Verfahren ein Extrahieren des Holzmaterials mit einem organischen lipophilen Lösemittel wie etwa Kohlenwasserstoff ebenso wie mit einem organischen hydrophilen Lösemittel wie etwa eine organische niedere Alkylcarbonylverbindung umfasst, und dass die lipophile und/oder hydrophile Extraktionslösung einen Teil des Lösemittelsystems der Zusammensetzung des Trägermittels bildet.

10. Verwendung des Verfahrens nach Anspruch 1 für eine Herstellung eines Haarpflegeprodukts, das in Anspruch 1 definiert ist, wobei das Verfahren ein Extrahieren des Holzmaterials mit einem monovalenten oder multivalenten Alkohol oder deren Mischung umfasst, wobei die Extraktionslösung vorzugsweise der multivalente, niedere Akylalkohol ist, der vorteilhaft aus den folgenden Gruppen ausgewählt ist: niederer Akylaldiol wie etwa Propylglykol, Butylglykol, Pentylglykol, Octylglykol, niederer Akylaltriol und deren Ester.

11. Verwendung des Verfahrens nach Anspruch 10 für eine Herstellung eines Haarpflegeprodukts, das in Anspruch 1 definiert ist, wobei das Verfahren ein Anwenden der Lösung umfasst, die ein niederes Alkan ist, vorzugsweise ein Hexan oder Heptan wie die Extraktionslösung des Holzmaterials.

12. Verwendung des Verfahrens nach Anspruch 9 für eine Herstellung eines Haarpflegeprodukts, das in Anspruch 1 definiert ist, wobei in dem Verfahren die Extraktionslösung und der Träger, der in der Zusammensetzung vorhanden ist, eine auf Kohlenwasserstoff basierende Lösung oder ein Keton oder eine anorganische Anion-Kation-Lösung oder eine ionogene Lösung oder einen Ester oder einen dreiwertigen Alkohol enthält.

## Revendications

1. Procédé comprenant les étapes suivantes :
- dans la composition, est inclus un agent de transport aqueux, soit de nature hydrophobe soit de nature hydrophile, choisi selon la cible d'utilisation et des agents tensioactifs, conventionnellement utilisés dans une composition cosmétique, des parfums et de possibles agents de dispersion, et
- en connexion avec la production du transporteur, est inclus un mélange de composés largement antimicrobien pour la quantité comprise entre 0,1 et 5% en poids à partir du poids total de ladite composition, dans lequel le mélange de composés obtenu en pulvérisant du matériau en bois et/ou en extrayant le matériau en bois possiblement pulvérisé, de sorte que ledit mélange contient au moins deux composés différents choisis parmi le groupe constitué de : ligands selon la formule générale IA 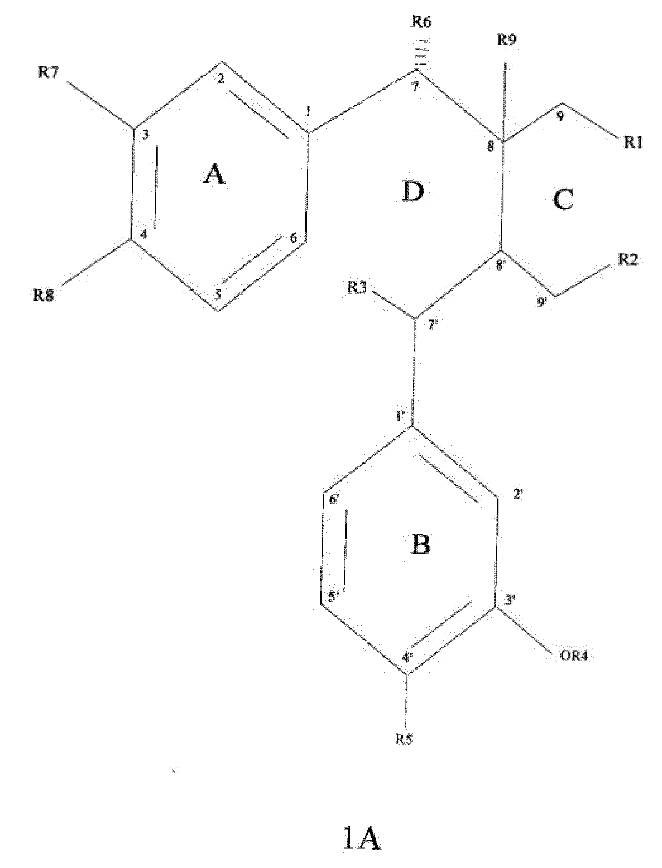 dans laquelle
R1 ou R2 désignent, indépendamment l'un de l'autre, un résidu choisi parmi les groupes hydrogène, OH ou =0,
ou soit un des résidus R1 ou R2 désigne la liaison de l'atome d'oxygène -0- aux carbones 9 et 9', et maintenant forme avec les carbones 8, 8', 9, 9' un noyau C hétérocyclique oxygéné à 5 chainons,
R3 désigne l'hydrogène ou un résidu choisi parmi le groupe OH, =0, ou il forme une liaison au carbone 6, de sorte que les carbones 6, 1, 7, 8, 8', 7' forment un cycle cyclohexane qui est condensé avec un noyau phényle A, et éventuellement aussi un noyau avec C,
R4 désigne l'hydrogène ou méthyle,
R5 désigne l'hydrogène ou un résidu choisi parmi les groupes OH et OCH₃,
R6 désigne l'hydrogène ou hydroxy,
R7 et R8 désignent, indépendamment l'un de l'autre, l'hydrogène ou un résidu choisi parmi les groupes OH et OCH₃,
R9 désigne H ou en cas de nortrachélogénine R9 désigne OH
ou des ligands de formule générale IB 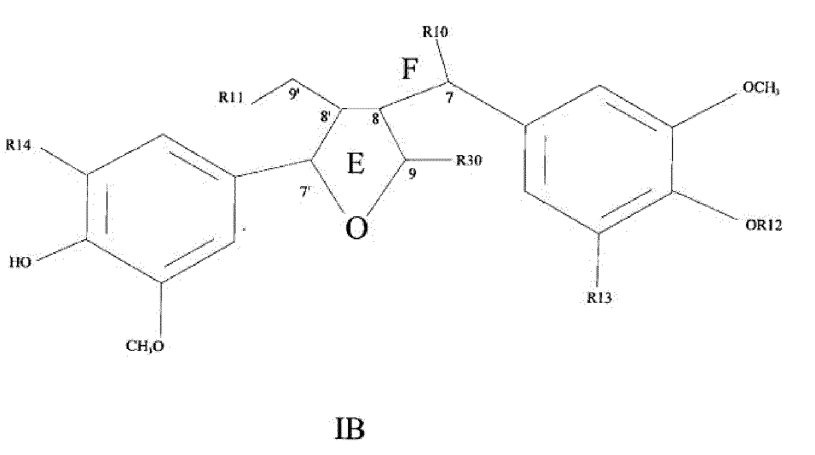 dans laquelle
R10 désigne l'hydrogène ou hydroxy R11 désigne hydroxy ou l'oxygène, qui est lié par une liaison aux carbones 7 et 9', formant maintenant un noyau (tétrahydrofurane) F hétérocyclique à 5 chainons non aromatique oxygéné avec les carbones 7, 8, 8', 9', lequel noyau est condensé dans le noyau E (tétrahydrofurane) hétéro aux carbones 8, 8',
R12 désigne l'hydrogène ou méthyle,
R13 désigne l'hydrogène ou méthoxy,
R14 désigne l'hydrogène ou méthoxy,
R30 désigne l'hydrogène ou un groupe =0 et
des stilbènes selon la formule générale II 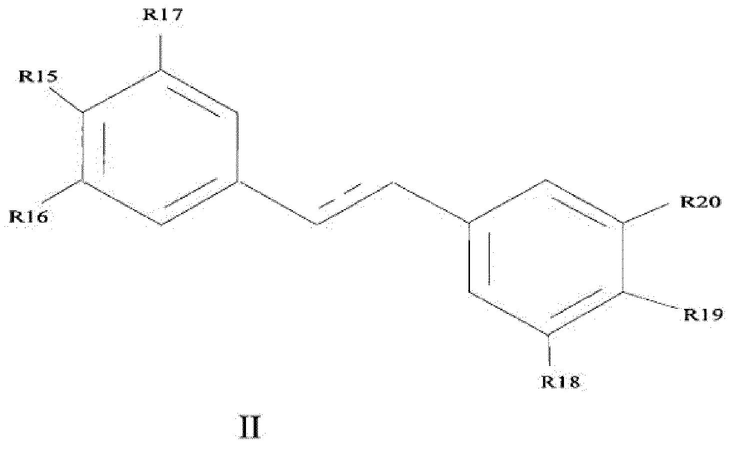 dans laquelle
R15 désigne l'hydrogène ou hydroxy
R16 désigne un résidu choisi parmi les groupes H, OH, OCH₃,
R17 désigne un résidu choisi parmi les groupes OH ou OCH₃,
R18 et R19 désignent, indépendamment l'un de l'autre, l'hydrogène ou hydroxy,
R20 désigne un résidu choisi parmi les groupes hydrogène, OGlu et des juvabiones selon la formule générale III, 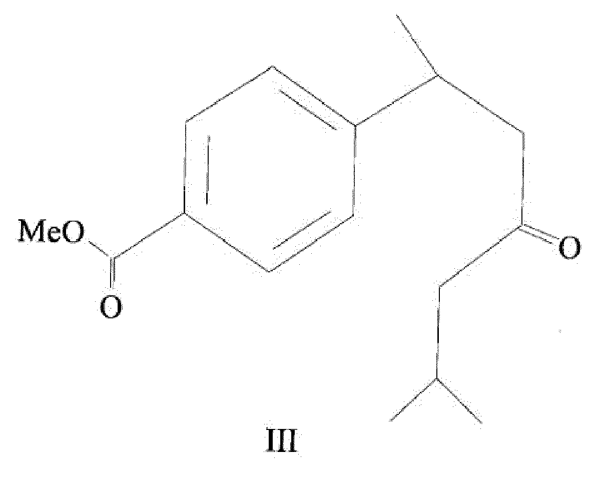
et des flavonoïdes selon la formule générale IV 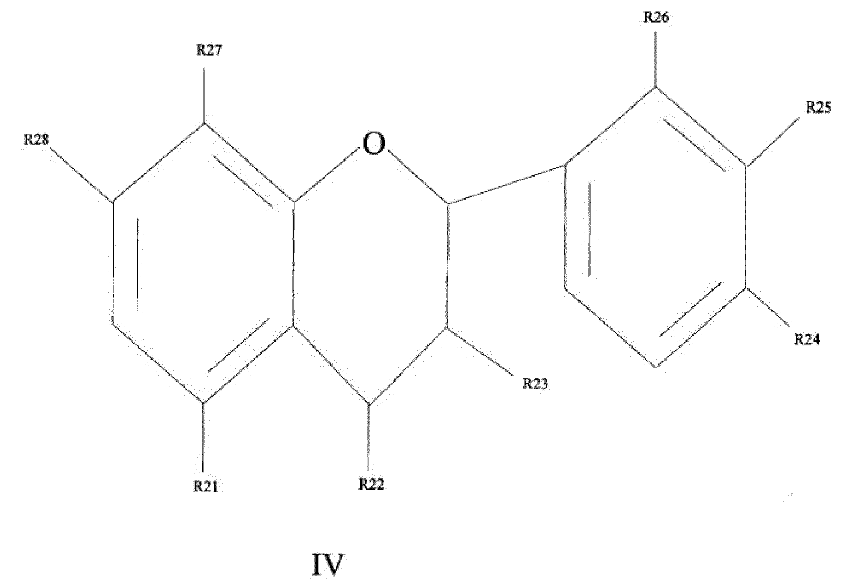 dans laquelle
R21 désigne un résidu choisi parmi les groupes H, OH, R22 désigne un résidu choisi parmi les groupes H, OH, =0,
R23 désigne un résidu choisi parmi les groupes H, OH,
R24, R25, R26 désignent, indépendamment l'un de l'autre, l'hydrogène ou hydroxy,
R28 et R27 désignent, indépendamment l'un de l'autre, l'hydrogène ou hydroxy, et la bétuline, l'acide bétulonique, l'acide bétulinique, l'acide bétuloïnique, ainsi que leur dérivés ester et éther, ainsi que des stéréoisomères ; ledit mélange de composés contenant en outre des oligomères desdits composés, à condition, cependant, que le mélange contient des ligands, en particulier 7-hydroxymatairésinol ou sécoisolaricirésinol pour de 50 à 99,9% en poids, des stilbènes, en particulier la pinosylvine ou ses dérivés éther ou ester pour de 0 à 70% en poids, des oligomères de ligands, des stilbènes, des juvabiones ou des flavonoides pour 31% en poids, à condition en outre que le mélange contient au moins un composé choisi parmi le groupe suivant : 7-hydroxymatairésinol, conidendrine, acide conidendrique, alpha-conidendrine, acide alpha-conidendrique, isohydroxymatairésinol, cycloaricirésinol, sécoisolaricirésinol, anhydrosécoisolaricirésinol et des stilbènes, ainsi que leur dérivés éther et ester et stéréoisomères, dans lequel ledit procédé la quantité dudit mélange de composés est maintenu dans une telle dite quantité,
- les composés contenus dans le mélange de composés n'irritent pas la peau chez un soi-disant test épicutané unique, et
- les composés contenus dans le mélange de composés sont tels que la cytotoxicité desdits composés, telle que mesurée dans de l'éthanol avec une culture cellulaire HaCat après 24 incubations, est inférieure à la cytotoxicité de 0,02 à 0,1% en poids de BHT dissout dans de l'éthanol dans les mêmes conditions d'incubation, de préférence inférieure à la cytotoxicité de 0,01 à 0,05% en poids de BHT dissout dans de l'éthanol dans les mêmes conditions d'incubation, et
- le mélange de composés possède également dans la composition un effet antimicrobien à grande échelle contre les bactéries à Gram-, les bactéries à Gram+, les levures et champignons dans lequel ledit effet antimicrobien à grande échelle signifie que le mélange de composés possède des effets pour inhiber, parmi les bactéries à Gram négatif, au moins la croissance de E. coli, Ps. Aeruginosa, Ps. Putida, KI. Pneimoniae ; parmi les bactéries à Gram positif, au moins la croissance de S. aureus ; parmi les levures au moins la croissance de levure provoquant des pellicules, M. furfur et C. albicans ; et parmi les champignons au moins la croissance de A. niger ;
pour la production d'un produit d'entretien des cheveux, y compris d'un agent de transport aqueux ; un mélange de composés largement antimicrobien mentionné ci-dessus, qui inhibe la croissance des levures provoquant des pellicules, Malassezia furfur ; des agents tensioactifs, des parfums et de possibles agents de dispersion.

2. Utilisation du procédé selon la revendication 1 pour la production d'un produit d'entretien des cheveux défini dans la revendication 1 qui contient en outre un agent antimicrobien à petite échelle.

3. Utilisation du procédé selon la revendication 1 ou 2 pour la production d'un produit d'entretien des cheveux, défini dans la revendication 1, dans laquelle dans le mélange de composés les oligomères sont des oligolignans.

4. Utilisation du procédé selon l'une quelconque des revendications précédentes pour la production d'un produit d'entretien des cheveux, défini dans la revendication 1, dans laquelle le procédé consiste à obtenir le mélange de composés en pulvérisant le matériau en bois à partir d'au moins deux espèces de bois différentes et/ou en extrayant du matériau en bois possiblement pulvérisé à partir de deux espèces de bois différentes, de sorte que le matériau en bois est de préférence du matériau en noeuds de bois ou du matériau en bois de fût d'adjacent aux noeuds, et que lors du procédé d'extraction, on emploie avantageusement un solvant hydrophile organique, de préférence un solvant organique à base d'hydrocarbure-carbonyle spécialement une solution contenant des alkyl carbonyles tels que des alcools ou des cétones.

5. Utilisation du procédé selon la revendication 4 pour la production d'un produit d'entretien des cheveux, défini dans la revendication 1, dans laquelle dans le procédé le mélange de composés est obtenu en extrayant le matériau en bois de pin et d'épicéa, et que le mélange de composés contient à la fois des stilbènes et de lignans, de 50 à 99,9% des lignans étant obtenus à partir du matériau en noeuds d'épicéa ou du matériau du bois de fût d'épicéa adjacent aux noeuds, et de 0,1 à 50% des lignans est obtenu à partir du matériau en noeuds de pin ou du matériau en bois de fût de pin adjacent aux noeuds.

6. Utilisation du procédé selon l'une quelconque des revendications précédentes pour la production d'un produit d'entretien des cheveux, défini dans la revendication 1, dans laquelle le procédé comprend l'inclusion d'un agent antimicrobien à petite échelle qui est de préférence la bétuline, l'acide bétulonique, l'acide bétulinique, l'acide bétuloïnique ou leur dérivés obtenus à partir de l'écorce de bouleau, ou du resvératrol ou son dérivé obtenu à partir de l'écorce d'épicéa, ou du glycéryle d'éthylhexyle.

7. Utilisation du procédé selon l'une quelconque des revendications 1 a 6 pour la production d'un produit d'entretien des cheveux, défini dans la revendication 1, dans laquelle le procédé comprend l'inclusion en tant qu'additifs, des agents antioxydants tels que des vitamines naturelles ou synthétiques, par exemple la vitamine A, B, C, D, E, la provitamine B5, la vitamine B3, l'acide L-ascorbique, la vitamine E, les antioxydants obtenus à partir de plantes, tels que les antioxydants contenus dans le thé vert, les antioxydants contenus dans la graine de lin, les antioxydants contenus dans le marron d'Inde, les béta carotènes, le sélénium, la glutamine, l'ubiquinone, l'acide glycolique, les hormones de croissance et la kinétine.

8. Utilisation du procédé selon l'une quelconque des revendications précédentes pour la production d'un produit d'entretien des cheveux, défini dans la revendication 1, dans laquelle le procédé comprend l'inclusion du mélange de composés qui contient au moins deux composés différents, choisis parmi le groupe suivant :
Lignans :
- matairésinol, hydroxymatairésinol, oxomatairésinol, didéméthyl matairésinol, isohydroxymatairésinol, épiisohydroxymatairésinol et leur stéréoisomères, parmi lesquels laissez nous signaler en particulier les stéréoisomères 7S, 8R de l'hydroxymatairésinol ; le 8'R-hydroxymatairésinol et 7R, 8R, 8'R-allohydroxymatairésinol, et leur stéréoisomères et dérivés ester ou éther,
- sécoisolaricirésinol, isolaricirésinol, laricirésinol, pinorésinol, diméthyl sécoisolaricirésinol, 7-hydroxysécoisolaricirésinol, cyclolaricirésinol, et leur stéréoisomères et dérivés ester ou éther,
- nortrachélogénine et ses stéréoisomères et dérivés ester ou éther,
- entérolactone et ses stéréoisomères et dérivés ester ou éther,
- conidendrine, α-conidendrine, et leur stéréoisomères et dérivés ester ou éther,
- lignan A et ses stéréoisomères et dérivés ester ou éther,
- liovile et ses stéréoisomères et dérivés ester ou éther ;
Juvabiones :
- juvabiones et leur stéréoisomères et dérivés ester ou éther,
Stilbènes :
- pinosylvine, dihydropinosylvine, éther monométhylique de pinosylvine, éther monométhylique de dihydropinosylvine, resvératrol, astringine, isorhapontine, et leur stéréoisomères et dérivés ester ou éther,
Flavonoïdes :
- pinosembrine, catéchine, pinobanxine, kaempférol, dihydrokaempférol, taxofoline, naringénine, téracasidine, kétotéracasidine, isotéracasidine, mélacasidine, isomélacasidine et leur stéréoisomères et dérivés ester ou éther,
- bétuline, acide bétulonique, acide bétulinique, acide bétuloïnique ou leur dérivés ester,
ainsi que les formes glycosidées de ces composés polyphénoliques et leur oligomères, tels que trimères et tétramères.

9. Utilisation du procédé selon la revendication 1 pour la production d'un produit d'entretien des cheveux, défini dans la revendication 1, dans laquelle le procédé comprend l'extraction du matériau en bois avec un solvant lipophile organique, tel qu'un hydrocarbure, ainsi qu'avec un solvant hydrophile organique, tel qu'un composé alkyl carbonyle inférieur organique, et que la solution d'extraction lipophile et/ou hydrophile fait partie de l'agent de transport de la composition.

10. Utilisation du procédé selon la revendication 1 pour la production d'un produit d'entretien des cheveux, défini dans la revendication 1, dans laquelle le procédé comprend l'extraction du matériau en bois avec un alcool monovalent ou multivalent ou leur mélange dans lequel la solution d'extraction est de préférence l'alkyl alcool inférieur multivalent qui est avantageusement choisi parmi les groupes suivants : un alkyl diode inférieur, tel que le glycol propylique, le glycol butylique, le glycol pentylique, le glycol octylique, un alkyl triol inférieur, et leurs esters.

11. Utilisation du procédé selon la revendication 10 pour la production d'un produit d'entretien des cheveux, défini dans la revendication 1, dans laquelle le procédé comprend l'emploi de la solution qui est un alcane inférieur, de préférence hexane ou heptane en tant que solution d'extraction du matériau en bois.

12. Utilisation du procédé selon la revendication 9 pour la production d'un produit d'entretien des cheveux, défini dans la revendication 1, dans laquelle dans le procédé la solution d'extraction et le transporteur présent dans la composition contient une solution à base d'hydrocarbures, ou une cétone, ou une solution anion-cation inorganique ou une solution ionogène, ou un ester d'un alcool trivalent.
